# EUROPEAN PATENT SPECIFICATION

(11) **EP 3 324 950 B1**
(45) Date of publication and mention of the grant of the patent: **15.01.2020**
(21) Application number: 16745509.6
(22) Date of filing: 21.07.2016
(51) Int. Cl.: A61K 31/00, A61K 31/4439, A61K 31/472, A61K 31/4725, A61K 31/55, A61P 17/00

(54) **TREATMENT FOR VITILIGO**
BEHANDLUNG DER WEISSFLECKENKRANKHEIT
TRAITEMENT DU VITILIGO

(30) Priority: 24.07.2015 US 201562196349 P
(43) Date of publication of application: 30.05.2018
(73) Proprietor: Glaxo Group Limited, Brentford, Middlesex TW8 9GS (GB)
(72) Inventor: DICKSON, Marion Catherine, Stevenage Hertfordshire (GB); RUBENSTEIN, David, Durham, North Carolina 27709 (US)
(74) Representative: Hillier, Mark
(86) International application number: PCT/IB2016/054355
(87) International publication number: WO 2017/017571

(56) References cited:
- US-A1- 2012 232 061
- US-A1- 2014 073 659

## Description

### FIELD OF THE INVENTION

The present disclosure relates to use of spleen tyrosine kinase (Syk) compounds, their hydrates, solvates, or pharmaceutically salts thereof, and pharmaceutical compositions comprising a SYK compound and methods of using these compounds and compositions thereof in the treatment of vitiligo.

### BACKGROUND

Vitiligo is an autoimmune disease of the skin that results in disfiguration of the skin, generally with white spots. The condition causes depigmentation of skin, typically in sections or patches, and affects about 0.5-2% of the world population. Vitiligo occurs when there is an absence of functional melanocytes (melanin-producing cells) in the skin. Vitiligo also can affect the mucous membranes and the eye. There may be a genetic predisposition to vitiligo in some cases. The average age at vitiligo onset is about 20 years, with onset most commonly observed between the ages of 10 and 30.

Vitiligo patients have increased numbers of autoreactive, melanocyte-specific CD8+ T cells in the skin and blood, which are directly responsible for melanocyte destruction. T cells infiltrate the skin during vitiligo and localize to the epidermis where melanocytes, their target cells, reside. In lesional skin, CD8+ T cells are found in close proximity to dying melanocytes and one study reported that melanocytespecific, CD8+ T cells isolated from lesional skin migrated into nonlesional skin ex vivo, found their melanocyte targets in situ, leading to cell death. The melanocyte niche in the basal layer of the epidermis is not vascularized, so T cells that migrate into the skin in vitiligo must efficiently navigate through the dermis to find their targets and mediate depigmentation. The signals that promote melanocytespecific T cell migration into and through the skin during vitiligo are unknown. There is recent evidence that suggests the chemokine CXCL0 may be fundamental to this process (see Rashighi et al., Science Translational Medicine 2014, Feb. 12, Vol. 6, Issue 223, pp 223ra23).

The exact cause of the melanocyte loss in vitiligo remains debatable, but recent observations have pointed to a role for cellular immunity in the pathogenesis of vitiligo (see, for example, Wang et al. (2011) Th17 Cells and Activated Dendritic Cells Are Increased in Vitiligo Lesions. PLoS ONE 6(4): e18907).

Vitiligo occurs most often on the face and extremities-typically the hands and wrists. Depigmentation also can occur around the mouth, eyes, nostrils, genitalia, and umbilicus. Depigmented patches are flat areas of normal-feeling skin, and may have a hyperpigmented edge. The edges typically are well-defined but irregular. In trichrome vitiligo, there is an intermediate zone of hypochromia between the achromic center and peripheral unaffected skin.

There are several clinical classifications of vitiligo. Segmental vitiligo presents as one or more macules in a dermatomal or quasidermatomal pattern, and occurs most commonly in children. All other types of vitiligo are classified as non-segmental vitiligo, which is most common. Focal vitiligo is characterized by depigmentation in one area, or macule, such as the trigeminal nerve distribution. Other forms of non-segmental vitiligo often produce symmetric patches, sometimes covering large areas. Mucosal vitiligo affects only mucosal membranes. Generalized vitiligo may be acrofacial, in which depigmentation occurs on the distal fingers and periorificial areas, or vulgaris, which is characterized by widely distributed, scattered patches. Universal vitiligo manifests as complete or nearly complete depigmentation, and frequently is associated with multiple endocrinopathy syndromes.

US Publication 2014/0073659, Magilavy, D., Rigel Pharmaceuticals, describe compounds which are inhibitors of SYK kinases and which are useful for the treatment of vitiligo.

There is currently no U.S. Food and Drug Administration approved medical treatment for vitiligo, and available off-label treatments are problematic and often ineffective. Current therapies include use of topical corticosteroids, topical immunomodulators such as calcineruin inhibitors, and psoralen phototherapy, including narrow-band ultraviolet B (nbUVB) light. Vitiligo is a disfiguring disease for which current therapies have proven unsatisfactory.
Therefore, the identification of a targeted therapy, and one having a good safety profile would be a substantial advance for treatment in this area.

### BRIEF DESCRIPTION OF THE DRAWINGS

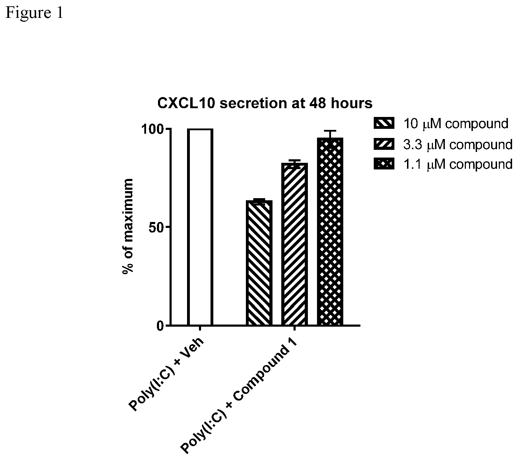

Figure 1 demonstrates a dose-dependent decrease in CXCL10 with a compound of Formula (1) in a poly(I:C)-induced CXCL10 secretion assay in HaCaT cells.

### SUMMARY OF THE INVENTION

The present invention is directed to a method of treating, including prophylactic treatment, of a subject who is at risk, or has developed vitiligo, with a pharmaceutically effective amount of a compound of Formula (I) 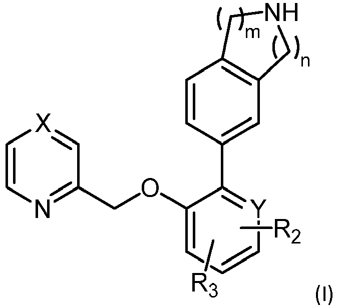 wherein:
X is CR₁ or N;
Y is CH, C or N;
R₁ is hydrogen, C₁₋₆alkoxy or C₁₋₆alkyl;
R₂ is hydrogen, C₁₋₆alkoxy, halo, -C(O)C₁₋₆alkyl, CN, halo-C₁₋₆alkyl or -C(O)NR₄R₅;
R₃ is hydrogen or C₁₋₆alkoxy;
R₄ is hydrogen or C₁₋₆alkyl:
R₅ is hydrogen or C₁₋₆alkyl; and
m and n are integers each independently selected from 1 and 2, or
a pharmaceutically acceptable salt thereof.

Another embodiment of the invention is directed to a method of treating a subject who has developed vitiligo, with a pharmaceutically effective amount the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

Another embodiment of the invention is directed to a method of treating a subject who is at risk of developing vitiligo with a pharmaceutically effective amount the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

Another embodiment of the invention is directed to a method of treating a subject who has developed vitiligo, with a pharmaceutically effective amount the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof.

Another embodiment of the invention is directed to a method of treating a subject who has developed vitiligo, with a pharmaceutically effective amount the compound 7-(2-(methyloxy)-6-{[(4-methyl-2-pyridinyl)methyl]oxy}phenyl)-2,3,4,5-tetrahydro-1*H*-3-benzazepine, mesylate.

Another embodiment of the invention is directed to a method of treating a subject who is at risk for developing vitiligo with a pharmaceutically effective amount the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof. In one embodiment, the pharmaceutically acceptable salt is the mesylate salt.

The compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof may be administered topically, or ocularly. In one embodiment, the compound is applied directly to depigmented areas of skin. In another embodiment, the compound is applied to the eyelid of said patient.

The compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof may be administered topically, or ocularly. In one embodiment, the pharmaceutically acceptable salt is the mesylate salt. In one embodiment, the compound is applied directly to depigmented areas of skin. In another embodiment, the compound is applied to the eyelid of said patient.

Another aspect of the invention is use of a pharmaceutical composition for treating vitiligo by administering to the subject an effective amount of a compound of Formula (I), or a pharmaceutically acceptable salt thereof and a pharmaceutically acceptable carrier adapted for topical administration or delivery to the skin or ocular area of a patient in need thereof.

Another aspect of the invention is use of a pharmaceutical composition for treating vitiligo by administering to the subject an effective amount of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof and a pharmaceutically acceptable carrier adapted for topical administration or delivery to the skin or ocular area of a patient in need thereof. In one embodiment, the pharmaceutically acceptable salt is the mesylate salt.

Another aspect of the invention is use of a pharmaceutical composition for treating vitiligo by administering to the subject an effective amount of the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof and a pharmaceutically acceptable carrier adapted for topical administration or delivery to the skin or ocular area of a patient in need thereof.

Another aspect of the invention is directed to a method of treating, including prophylactic treatment, of a subject who is at risk, or has developed vitiligo, with a pharmaceutically effective amount of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof. In one embodiment, the pharmaceutically acceptable salt is the mesylate salt.

Another aspect of the invention is directed to a method of treating, including prophylactic treatment, of a subject who is at risk, or has developed vitiligo, with a pharmaceutically effective amount of the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

A compound of Formula (I), or a pharmaceutically acceptable salt thereof may be administered topically, or ocularly. In another embodiment a composition comprising a compound of Formula (I), or a pharmaceutically acceptable salt thereof and a pharmaceutically acceptable carrier is administered topically, or ocularly. In one embodiment, the compound or composition of Formula (I) is applied directly to depigmented areas of skin. In another embodiment, the compound or composition of Formula (I) is applied to the eyelid of said patient.

In one embodiment the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof which may be administered topically, or ocularly. In another embodiment there is a composition comprising the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof and a pharmaceutically acceptable carrier may be administered topically, or ocularly. In one embodiment, the compound is applied directly to depigmented areas of skin. In another embodiment, the compound is applied to the eyelid of said patient.

Another aspect of the invention is use of a pharmaceutical composition for treating vitiligo by administering to the subject an effective amount of a compound of Formula (I), or a pharmaceutically acceptable salt thereof and a pharmaceutically acceptable carrier adapted for topical administration or deliver to the skin or ocular area of a patient in need thereof.

Another aspect of the invention is use of a pharmaceutical composition for treating vitiligo by administering to the subject an effective amount of the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof and a pharmaceutically acceptable carrier adapted for topical administration or deliver to the skin or ocular area of a patient in need thereof.

Another aspect of the invention is use of a pharmaceutical composition for treating vitiligo by administering to the subject an effective amount of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof and a pharmaceutically acceptable carrier adapted for topical administration or deliver to the skin or ocular area of a patient in need thereof.

In one embodiment the compound is administered in a topical formulation directly to depigmented skin, or localized areas including depigmented patches (e.g., the hands or face), without applying it to any substantial amount of unaffected skin. In another embodiment the compound is administered in a topical formulation directly to depigmented skin, or localized areas including depigmented patches (e.g., the hands or face), and to unaffected skin surrounding the depigmented skin.

In another embodiment, the topical administration of a compound of Formula (I), or a pharmaceutically acceptable salt thereof is effective to cause at least partial regression, such as repigmentation of existing areas and/or reduced incidence of new areas, of the depigmented skin that characterize the disease.

In another embodiment, the topical administration of the compound, 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof is effective to cause at least partial regression, such as repigmentation of existing areas and/or reduced incidence of new areas, of the depigmented skin that characterize the disease.

In another embodiment, the topical administration of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof is effective to cause at least partial regression, such as repigmentation of existing areas and/or reduced incidence of new areas, of the depigmented skin that characterize the disease. In one embodiment, the pharmaceutically acceptable salt is the mesylate salt.

In another embodiment the administration of a compound of Formula (I), or a pharmaceutically acceptable salt thereof is effective to prevent vitiligo in a subject at risk of developing vitiligo.

In another embodiment the administration of compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof is effective to prevent vitiligo in a subject at risk of developing vitiligo.

In another embodiment the administration of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof is effective to prevent vitiligo in a subject at risk of developing vitiligo. In one embodiment, the pharmaceutically acceptable salt is the mesylate salt.

In one embodiment, the subject is first determined to have vitiligo, for example the subject may display one or more clinical and/or histopathological features of vitiligo. In another embodiment, the subject is first determined to be at risk of developing vitiligo. For example, the subject may have a family history of vitiligo and be genetically at risk of developing vitiligo, and/or the subject may have a history of a disease associated with vitiligo, such as thyroid disease. Thus there is potential for use of a compound of Formula (I) for prophylatic or for maintenance therapy in an individual who has active disease that had been treated and undergone disease regression.

Another embodiment of the invention, the vitiligo of a subject in need thereof, is treated using an effective amount of a compound of formula (I), or a pharmaceutically acceptable salt thereof in combination or adjunctively with a second therapeutic treatment and/or second therapeutic active agent.

Another embodiment of the invention, the vitiligo of a subject in need thereof, is treated using an effective amount of the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof in combination or adjunctively with a second therapeutic treatment and/or second therapeutic active agent.

Another embodiment of the invention, the vitiligo of a subject in need thereof, is treated using an effective amount of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof in combination or adjunctively with a second therapeutic treatment and/or second therapeutic active agent. In one embodiment, the pharmaceutically acceptable salt is the mesylate salt.

In one embodiment the second therapeutic active agent is an anti-inflammatory compound, an antihistamine compound, an antibiotic compound, an antiviral compound, antifungal, thyroid hormone replacement compounds, or any combination thereof.

In another embodiment the second therapeutic treatment is irradiation, systemic phototherapy, psoralen photochemotherapy, or excimer laser therapy.

In yet another embodiment the second therapeutic treatment or second therapeutic agent may both be utilized together in combination with the compound.

In another aspect of the invention the compound or a pharmaceutical composition comprising the compound and a carrier, is administered either alone or in combination or adjunctively with one or more additional therapeutic agents.

In another aspect of the invention a compound of Formula (I), or a pharmaceutical composition comprising a compound of Formula (I) and a pharmaceutically acceptable carrier is administered alone or with another pharmaceutically active agent, and with a topical sunscreen filter (to minimize the exposure to ultraviolet light that may worsen vitiligo, cause sunburn, or cause skin cancer of depigmented areas). Yet other combination treatments can include the use of concomitant or adjunctive treatment including those treatments described above.

In another aspect of the invention the compound of Formula (I), or a pharmaceutical composition comprising a compound of Formula (I), and a pharmaceutically acceptable carrier when used for treating vitiligo, is administered at least once daily, or it is applied more than once daily, such as at least two, three or four times daily. In one embodiment the formulation is applied to the skin in a sustained release formulation for release of the compound. In one embodiment the compound of Formula (I) is 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof. In another embodiment the compound of Formula (I) is compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof.

### DETAILED DESCRIPTION OF THE INVENTION

Syk is a non-receptor tyrosine kinase involved in coupling activated immunoreceptors to signal downstream events that mediate diverse cellular responses, including proliferation, differentiation, and phagocytosis. Syk is widely expressed in hematopoietic cells. Syk inhibitors have potential anti-inflammatory and immunomodulating activities. They inhibit Syk-mediated IgG Fc epsilon and gamma receptor and BCR receptor signalling, resulting in inhibition of the activation of mast cells, macrophages, and B-cells and related inflammatory responses and tissue damage. Mast cells play a major role in type I hypersensitivity reactions and have been implicated in urticaria, bronchial asthma, anaphylaxis and other allergic conditions. Accordingly, Syk inhibitors have attracted interest in a number of therapeutic areas, including the treatment of rheumatoid arthritis, B-cell lymphoma, asthma, rhinitis and cutaneous disorders such as acute and chronic urticaria, mastocytosis, cutaneous lupus, atopic dermatitis, autoimmune bullous conditions including pemphigus and pemphigoid and other mast cell mediated diseases of the skin.

It has been reported that gene expression in lesional skin from vitiligo patients reveals an interferon-g (IFN-g)-specific signature, including the chemokine CXCL10. CXCL10 was found to be elevated in both vitiligo patient skin and serum, and CXCR3, its receptor, was expressed on pathogenic T cells [Rahighi et al., Sci Transl Med., 6, 223ra23 (2014)]. Data generated in the Rahigi et al study identify a critical role for CXCL10 in both the progression and maintenance of vitiligo. CXCL10 is highly expressed in the skin and serum of patients with vitiligo, and it promotes autoreactive T cell localization to the epidermis to initiate vitiligo in their mouse model. Targeting CXCL10 therapeutically in mice not only prevented disease development but also induced repigmentation in established lesions, implicating this chemokine in both the progression and maintenance of vitiligo.

Epidermal keratinocytes are a major source of CXCL10, the chemoattractant for the melanocyte specific autoreactive T-cells in vitiligo. Blocking SYK is believed to inhibit keratinocyte CXCL10 production, thus providing another therapeutic route to treat vitiligo.

Compounds of Formula (I) are believed to inhibit CXCL10 production, thus opening up a new group of therapeutic areas for treatment, including but not limited to vitiligo.

Compounds of Formula (I) have the following structure: 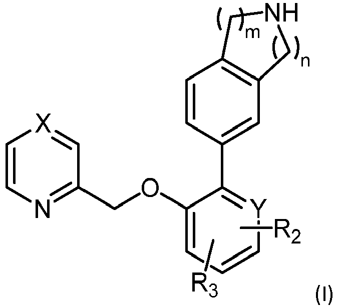 wherein:
X is CR₁ or N;
Y is CH, C or N;
R₁ is hydrogen, C₁₋₆alkoxy or C₁₋₆alkyl;
R₂ is hydrogen, C₁₋₆alkoxy, halo, -C(O)C₁₋₆alkyl, CN, Halo-C₁₋₆alkyl or -C(O)NR₄R₅;
R₃ is hydrogen or C₁₋₆alkoxy;
R₄ is hydrogen or C₁₋₆alkyl:
R₅ is hydrogen or C₁₋₆alkyl; and
m and n are integers each independently selected from 1 and 2, or a pharmaceutically acceptable salt thereof.

In one embodiment, the invention provides a compound of Formula (Ia): 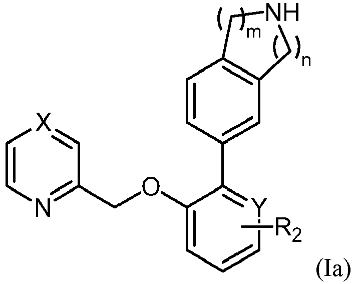 wherein:
X is CR₁ or N;
Y is CH, C or N;
R₁ is hydrogen, C₁₋₆alkoxy or C₁₋₆alkyl;
R₂ is hydrogen, C₁₋₆alkoxy, halo or -C(O)C₁₋₆alkyl; and
m and n are integers each independently selected from 1 and 2; or
a salt thereof.

In one embodiment X is CR₁ or N. In another embodiment X is CR₁.

In one embodiment Y is CH, C or N. In another embodiment Y is CH. In another embodiment Y is C. In a further embodiment Y is N.

In one embodiment R₁ is hydrogen, C₁₋₆alkoxy or C₁₋₆alkyl. In another embodiment R₁ is methyl, methoxy or hydrogen.

In one embodiment R₂ is hydrogen, C₁₋₆alkoxy, halo, -C(O)C₁₋₆alkyl, CN, Halo-C₁₋₆₋alkyl or C(O)NR₄R₅. In one embodiment R₂ is hydrogen, C₁₋₆alkoxy, halo or -C(O)C₁₋₆alkyl. In another embodiment R₂ is hydrogen, methoxy, fluoro, -C(O)CH₃ or trifluoromethyl. In another embodiment R₂ is hydrogen, methoxy, fluoro or -C(O)CH₃. In a further embodiment R₂ is hydrogen, methoxy or -C(O)CH₃.

In one embodiment R₃ is hydrogen or C₁₋₆alkoxy. In one embodiment R₃ is hydrogen, or methoxy.

In one embodiment R₄ is hydrogen or C₁₋₆alkyl. In another embodiment R₄ is hydrogen or C₁₋₄alkyl. In a further embodiment R₄ is hydrogen or methyl.

In one embodiment R₅ is hydrogen or C₁₋₆alkyl. In another embodiment R₅ is hydrogen or C₁₋₄alkyl. In a further embodiment R₅ is hydrogen or methyl.

In one embodiment m and n are integers each independently selected from 1 and 2. In another embodiment m is 2 and n is 1 or 2. In another embodiment n is 1 and m is 1 or 2. In a further embodiment m and n are both 2.

In one embodiment X is CR₁, and R₁ is methyl. In another embodiment, X is CR₁, R₁ is methyl and Y is C. In a further embodiment, X is CR₁, R₁ is methyl, Y is C and R₂ is methoxy. In a yet further embodiment, X is CR₁, R₁ is methyl, Y is C, R₂ is methoxy and R₃ is hydrogen. In a still further embodiment, X is CR₁, R₁ is methyl, Y is C, R₂ is methoxy, R₃ is hydrogen, m is 2 and n is 2.

In one embodiment, the compound of formula (I) is selected from: 7-
(3-{[(4-methyl-2-pyridinyl)methyl]oxy}-2-pyridinyl)-2,3,4,5-tetrahydro-1H-3-benzazepine;
7-(2-(methyloxy)-6-{[(4-methyl-2-pyridinyl)methyl]oxy}phenyl)-2,3,4,5-tetrahydro-1H-3-benzazepine;
7-(4-(methyloxy)-2-{[(4-methyl-2-pyridinyl)methyl]oxy}phenyl)-2,3,4,5-tetrahydro-1H-3-benzazepine;
1-[4-{[(4-methyl-2-pyridinyl)methyl]oxy}-3-(2,3,4,5-tetrahydro-1H-3-benzazepin-7-yl)phenyl]ethanone;
7-(6-methyl-3-{[(4-methyl-2-pyridinyl)methyl]oxy}-2-pyridinyl)-2,3,4,5-tetrahydro-1H-3-benzazepine;
1-[4-[(2-pyrazinylmethyl)oxy]-3-(2,3,4,5-tetrahydro-1H-3-benzazepin-7-yl)phenyl]ethanone;
7-(5-fluoro-2-{[(4-methyl-2-pyridinyl)methyl]oxy}phenyl)-1,2,3,4-tetrahydroisoquinoline;
7-(5-methyl-2-{[(4-methyl-2-pyridinyl)methyl]oxy}phenyl)-1,2,3,4-tetrahydroisoquinoline;
7-(2-(methyloxy)-6-{[(4-methyl-2-pyridinyl)methyl]oxy}phenyl)-1,2,3,4-tetrahydroisoquinoline;
7-(5-(ethyloxy)-2-{[(4-methyl-2-pyridinyl)methyl]oxy}phenyl)-1,2,3,4-tetrahydroisoquinoline;
7-(4-(methyloxy)-2-{[(4-methyl-2-pyridinyl)methyl]oxy}phenyl)-1,2,3,4-tetrahydroisoquinoline;
4-{[(4-methyl-2-pyridinyl)methyl]oxy}-3-(1,2,3,4-tetrahydro-7-isoquinolinyl)benzonitrile;
7-[2-{[(4-methyl-2-pyridinyl)methyl]oxy}-5-(trifluoromethyl)phenyl]-1,2,3,4-tetrahydroisoquinoline;
7-(5-(methyloxy)-2-{[(4-methyl-2-pyridinyl)methyl]oxy}phenyl)-1,2,3,4-tetrahydroisoquinoline;
7-(5-(1,1-dimethylethyl)-2-{[(4-methyl-2-pyridinyl)methyl]oxy}phenyl)-1,2,3,4-tetrahydroisoquinoline;
N-methyl-4-{[(4-methyl-2-pyridinyl)methyl]oxy}-3-(2,3,4,5-tetrahydro-1H-3-benzazepin-7-yl)benzamide;
4-{[(4-methyl-2-pyridinyl)methyl]oxy}-3-(2,3,4,5-tetrahydro-1H-3-benzazepin-7-yl)benzamide;
N,N-dimethyl-4-{[(4-methyl-2-pyridinyl)methyl]oxy}-3-(1,2,3,4-tetrahydro-7-isoquinolinyl)benzamide;
4-{[(4-methyl-2-pyridinyl)methyl]oxy}-3-(1,2,3,4-tetrahydro-7-isoquinolinyl)benzamide;
7-(2,3-bis(methyloxy)-6-{[(4-methyl-2-pyridinyl)methyl]oxy}phenyl)-1,2,3,4-tetrahydroisoquinoline;
7-(2,3-bis(methyloxy)-6-{[(4-methyl-2-pyridinyl)methyl]oxy}phenyl)-2,3,4,5-tetrahydro-1H-3-benzazepine;
7-[2-({[4-(methyloxy)-2-pyridinyl]methyl}oxy)phenyl]-2,3,4,5-tetrahydro-1H-3-benzazepine;
1-[4-[(2-pyridinylmethyl)oxy]-3-(2,3,4,5-tetrahydro-1H-3-benzazepin-7-yl)phenyl]ethanone, trifluoroacetate;
7-{6-methyl-3-[(2-pyrazinylmethyl)oxy]-2-pyridinyl}-2,3,4,5-tetrahydro-1H-3-benzazepine, trifluoroacetate;
7-(6-methyl-3-{[(4-methyl-2-pyridinyl)methyl]oxy}-2-pyridinyl)-2,3,4,5-tetrahydro-1H-3-benzazepine, trifluoroacetate;
7-{5-(methyloxy)-2-[(2-pyrazinylmethyl)oxy]phenyl}-2,3,4,5-tetrahydro-1H-3-benzazepine, trifluoroacetate;
7-[5-(methyloxy)-2-({[4-(methyloxy)-2-pyridinyl]methyl}oxy)phenyl]-2,3,4,5-tetrahydro-1H-3-benzazepine;
7-(5-(methyloxy)-2-{[(4-methyl-2-pyridinyl)methyl]oxy}phenyl)-2,3,4,5-tetrahydro-1H-3-benzazepine, trifluoroacetate;
1-[4-({[4-(methyloxy)-2-pyridinyl]methyl}oxy)-3-(2,3,4,5-tetrahydro-1H-3-benzazepin-7-yl)phenyl]ethanone;
1-[4-{[(4-methyl-2-pyridinyl)methyl]oxy}-3-(2,3,4,5-tetrahydro-1H-3-benzazepin-7-yl)phenyl]ethanone, trifluoroacetate;
7-[2-({[4-(methyloxy)-2-pyridinyl]methyl}oxy)phenyl]-2,3,4,5-tetrahydro-1H-3-benzazepine, trifluoroacetate;
1,1-dimethylethyl 7-[6-methyl-3-({[4-(methyloxy)-2-pyridinyl]methyl}oxy)-2-pyridinyl]-1,2,4,5-tetrahydro-3H-3-benzazepine-3-carboxylate;
7-(5-fluoro-2-{[(4-methyl-2-pyridinyl)methyl]oxy}phenyl)-2,3,4,5-tetrahydro-1H-3-benzazepine;
7-(5-methyl-2-{[(4-methyl-2-pyridinyl)methyl]oxy}phenyl)-2,3,4,5-tetrahydro-1H-3-benzazepine;
7-(5-(ethyloxy)-2-{[(4-methyl-2-pyridinyl)methyl]oxy}phenyl)-2,3,4,5-tetrahydro-1H-3-benzazepine;
7-(5-(methyloxy)-2-{[(4-methyl-2-pyridinyl)methyl]oxy}phenyl)-2,3,4,5-tetrahydro-1H-3-benzazepine;
4-{[(4-methyl-2-pyridinyl)methyl]oxy}-3-(2,3,4,5-tetrahydro-1H-3-benzazepin-7-yl)benzonitrile;
7-[2-{[(4-methyl-2-pyridinyl)methyl]oxy}-5-(trifluoromethyl)phenyl]-2,3,4,5-tetrahydro-1H-3-benzazepine;
7-(5-(1,1-dimethylethyl)-2-{[(4-methyl-2-pyridinyl)methyl]oxy}phenyl)-2,3,4,5-tetrahydro-1H-3-benzazepine;
7-(5-chloro-2-{[(4-methyl-2-pyridinyl)methyl]oxy}phenyl)-2,3,4,5-tetrahydro-1H-3-benzazepine;
7-(3-{[(4-ethyl-2-pyridinyl)methyl]oxy}-6-methyl-2-pyridinyl)-2,3,4,5-tetrahydro-1H-3-benzazepine;
7-(6-(1,1-dimethylethyl)-3-{[(4-methyl-2-pyridinyl)methyl]oxy}-2-pyridinyl)-2,3,4,5-tetrahydro-1H-3-benzazepine;
1-[4-{[(4-ethyl-2-pyridinyl)methyl]oxy}-3-(2,3,4,5-tetrahydro-1H-3-benzazepin-7-yl)phenyl]ethanone;
1-[4-({[4-(ethyloxy)-2-pyridinyl]methyl}oxy)-3-(2,3,4,5-tetrahydro-1H-3-benzazepin-7-yl)phenyl]ethanone;
1-[4-{[(4-{[2-(methyloxy)ethyl]oxy}-2-pyridinyl)methyl]oxy}-3-(2,3,4,5-tetrahydro-1H-3-benzazepin-7-yl)phenyl]ethanone;
7-{5-(methyloxy)-2-[(2-pyridinylmethyl)oxy]phenyl}-2,3,4,5-tetrahydro-1H-3-benzazepine;
1-[4-[(2-pyridinylmethyl)oxy]-3-(1,2,3,4-tetrahydro-7-isoquinolinyl)phenyl]ethanone; 7-{5-chloro-2-[(2-pyridinylmethyl)oxy]phenyl}-1,2,3,4-tetrahydroisoquinoline;
7-(6-chloro-3-{[(4-methyl-2-pyridinyl)methyl]oxy}-2-pyridinyl)-2,3,4,5-tetrahydro-1H-3-benzazepine;
7-(6-chloro-3-{[(4-methyl-2-pyridinyl)methyl]oxy}-2-pyridinyl)-1,2,3,4-tetrahydroisoquinoline;
1,1-dimethylethyl 5-{5-acetyl-2-[(2-pyridinylmethyl)oxy]phenyl}-1,3-dihydro-2H-isoindole-2-carboxylate;
1-[4-[(2-pyridinylmethyl)oxy]-3-(1,2,3,4-tetrahydro-6-isoquinolinyl)phenyl]ethanone; and
7-{2-(methyloxy)-6-[(2-pyrazinylmethyl)oxy]phenyl}-2,3,4,5-tetrahydro-1H-3-benzazepine, hydrochloride; or a salt thereof.

In one embodiment the compound of formula (I) is:
1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

In another embodiment the compound of formula (I) is 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof. In an embodiment, the pharmaceutically acceptable salt is a mesylate salt.

Compounds of Formula (I) may be prepared in accordance with the disclosure of Atkinson et al., US 2014/0005177 A1, USSN 14/004,388. The compound 7-(2-(methyloxy)-6-{[(4-methyl-2-pyridinyl)methyl]oxy}-phenyl)-2,3,4,5-tetrahydro-1*H*-3-benzazepine is specifically exemplified as Example 2 in US 2014/0005177 A1. Example 2a is specifically exemplifies the mesylate salt. The compound1-[4-{[(4-methyl-2-pyridinyl)methyl]oxy}-3-(2,3,4,5-tetrahydro-1*H*-3-benzazepin-7-yl)phenyl]ethanone is specifically exemplified as Example 4 in US2014/0005177 A1.

Another aspect of the invention embodiment, the present invention provides for the use of a compound of Formula (I) or a pharmaceutically acceptable salt thereof for the manufacture of a medicament for the treatment of a subject who is at risk, or has developed vitiligo. In another embodiment, the present invention provides for the use of the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof the manufacture of a medicament for the treatment, of a subject who is at risk, or has developed vitiligo. In another embodiment, the present invention provides for the use of the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof the manufacture of a medicament for the treatment, including prophylaxis, of a subject who is at risk, or has developed vitiligo.

A compound of Formula (I) for use in the treatment, including prophylaxis for treating vitiligo, is the compound 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof, the compound 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof. In one embodiment, the pharmaceutically acceptable salt thereof of the 7-(2-(methyloxy)-6-{[(4-methyl-2-pyridinyl)methyl]oxy}phenyl)-2,3,4,5-tetrahydro-1*H*-3-benzazepine is the mesylate salt.

In one aspect, the present invention provides for a compound of formula (I) or a pharmaceutically acceptable salt thereof, for use in the treatment of a dermatological n disease mediated by CXCL10.

### Abbreviations and Definition

As used herein, the following definitions or abbreviations shall apply unless otherwise indicated.

The term "alkyl" refers to a straight or branched saturated hydrocarbon chain containing the specified number of carbon atoms. For example, C₁₋₆alkyl means a straight or branched alkyl group containing at least 1, and at most 6, carbon atoms. Examples of "alkyl" as used herein include, but are not limited to, methyl, ethyl, n-propyl, n-butyl, n-pentyl, n-hexyl, isobutyl, isopropyl, t-butyl and 1,1-dimethylpropyl.

The term "alkoxy" refers to a straight or branched saturated alkoxy chain containing the specified number of carbon atoms. For example, C₁₋₆alkoxy means a straight or branched alkoxy group containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy or hexyloxy.

The term "halo" or, alternatively, "halogen" refers to fluoro, chloro or bromo.

The term "haloalkyl" refers to a straight or branched saturated hydrocarbon chain containing the specified number of carbon atoms, substituted with halo atoms. For example, Halo-C₁₋₆alkyl means a straight or branched alkyl group containing at least 1, and at most 6, carbon atoms, substituted with 1 to 3 halo atoms per carbon atom. Examples of "haloalkyl" as used herein include, but are not limited to, fluoromethyl, difluoromethyl, and tri-fluoromethyl.

The term "pharmaceutically acceptable" refers to those compounds, materials, compositions, and dosage forms which are, within the scope of sound medical judgment, suitable for use in contact with the tissues of human beings and animals without excessive toxicity, irritation, or other problems or complications, commensurate with a reasonable benefit/risk ratio. The skilled artisan will appreciate that pharmaceutically acceptable salts of the compound of Formula (I) may be prepared.

The term "pharmaceutically acceptable salts" refers to salts that retain the desired biological activity of the subject compound and exhibit minimal undesired toxicological effects. These pharmaceutically acceptable salts may be prepared *in situ* during the final isolation and purification of the compound, or by separately reacting the purified compound in its free acid or free base form with a suitable base or acid, respectively. Indeed, in certain embodiments of the invention, pharmaceutically acceptable salts may be preferred over the respective free base or free acid because such salts impart greater stability or solubility to the molecule thereby facilitating formulation into a dosage form.

As used herein, IL=Interleukin; STAT= Signal transducer and activator of transcription; SYK= Spleen Tyrosine Kinase.

The term "administering" refers to any method which delivers the composition to a patient in such a manner as to provide a therapeutic effect.

The term "Corticosteroids" are steroid structured based hormones. Corticosteroids are involved in a wide range of physiologic systems such as stress response, immune response and regulation of inflammation, carbohydrate metabolism, protein catabolism, blood electrolyte levels, and behavior. Representative examples include but are not limited to cortisol, prednisone and prednisolone, and fluticasone. Corticosteroids can be administered either orally, parenterally (for example by injection) or by direct topical application to a lesion on the skin, and they may be combined with the compound described herein as a combination formulation. These agents can be administered either orally, parenterally (for example by injection) or by direct topical application to an inflamed area, and they may be combined with the compound herein, or administered either contemporaneously, prior to or subsequent to the administration of the compound.

The term as used herein, "topical corticosteroids" are applied topically directly to the skin.

The term "cutaneous" or "dermal" refers to the skin. The skin consists of two layers (the dermis and epidermis), the outermost of which may be covered in many animals (including humans) at least in part with hair.

The term "dermatologically acceptable excipient" as used herein refers to any inactive ingredient present in the compositions described herein.

The term "external epithelial surfaces" are those exposed to the surfaces of the body (such as the skin, and the lining of the nose and mouth) and that are accessible to direct application of creams or ointments to the surface without the use of instrumentation (such as endoscopes or scalpels).

The term "non-steroidal anti-inflammatory drug (NSAID)" is a type of anti-inflammatory agent. Representative examples, include but are not limited to: ibuprofen, ketoprofen, piroxicam, naproxen, sulindac, aspirin, choline subsalicylate, diflunisal, fenoprofen, indomethacin, meclofenamate, salsalate, tolmetin and magnesium salicylate. These agents can be administered either orally, parenterally (for example by injection) or by direct topical application to an inflamed area, and they may be combined with the compound herein, or administered either contemporaneously, prior to or subsequent to the administration of the compound.

"Effective amount", "pharmaceutically effective amount" or "therapeutically effective amount" refers to any amount which will cause an improvement or change in the condition for which it is applied. The amount is sufficient to treat a disorder, disease or condition or one or more of its symptoms and/or to prevent the occurrence of the disease or disorder, and can be determined by standard clinical techniques. The amount may vary with the condition being treated, the stage of advancement of the condition, the body surface area affected with the clinical condition, and the type and concentration of formulation applied. Appropriate amounts in any given instance will be readily apparent to those skilled in the art or capable of determination by routine experimentation. The compositions are generally applied in topical manner to the affected area, i. e. localized application to the skin region where the clinical abnormality is manifest.

The term "subject" and/or "patient" refer to a human being of all ages, including pediatric, adult and geriatric patients.

The term "topical delivery" refers to application of a drug-containing formulation to the skin to directly treat cutaneous disorders or the cutaneous manifestations of a disease with the intent of substantially directing the pharmacological effect of the drug to the surface of the skin or within the skin.

The term 'topical administration' of the compound or the medicament refers to application to and absorption through the epithelial linings.

The term "applying" as used herein refers to any method which, in sound medical or cosmetic practice, delivers the topical composition to the lips of a subject in such a manner so as to provide a positive effect on a dermatological disorder, condition, or appearance.

The terms "modulate" or "modulates" refer to an increase or decrease in the amount, quality or effect of a particular activity.

The term 'biological agents' means complex biological molecules such as antibodies, monoclonal antibodies, proteins, polypeptides and nucleotides.

The term a 'treatment' for, or a 'method of treating', a medical condition, refers to a method of reducing, ameliorating or delaying the signs, symptoms, or progression of that medical condition, and includes prophylactic use thereof. As used herein, 'treatment' does not imply a cure. A treatment need not be effective in every member of a population, e.g., a population of patients with acne, to have clinical utility, as is recognized in the medical and pharmaceutical arts. As will be well understood in the art, "treatment" is an approach for obtaining beneficial or desired results, including clinical results. For the purposes of this invention, beneficial or desired results can include one or more, but are not limited to, alleviation or amelioration of one or more symptoms, diminishment of extent of a condition, including a disease, stabilized (i.e., not worsening) state of a condition, including diseases, preventing spread of disease, delay or slowing of condition, including disease, progression, amelioration or palliation of the condition, including disease, state, and remission (whether partial or total), whether detectable or undetectable. Treatment may also include arresting further advancement of a disease, as well as reversing in part the disorder, inducing regression of lesions.

It should be understood that the terms "a" and "an" as used above and elsewhere herein refer to "one or more" of the enumerated components. It will be clear to one of ordinary skill in the art that the use of the singular includes the plural unless specifically stated otherwise.

Throughout the application, descriptions of various embodiments use "comprising" language, however in some specific instances, an embodiment can alternatively be described using the language "consisting essentially of" or "consisting of".

The term "about" means within an acceptable range for the particular parameter specified as determined by one of ordinary skill in the art, which will depend, in part, on how the value is measured or determined, i.e., the limitations of the measurement system. For example, "about" can mean a range of up to 10% of a given value.

For the purposes of better understanding the present teachings and in no way limiting their scope, unless otherwise indicated, all numbers expressing quantities, percentages or proportions are to be understood as being modified in all instances by the term "about".

The term "vitiligo" is a condition characterized by loss of cutaneous melanocytes and/or abnormal melanocyte function.

Other terms used herein are intended to be defined by their well-known meanings in the art.
Concentrations, amounts, solubilities, and other numerical data may be presented herein in a range format. It is to be understood that such range format is used merely for convenience and brevity and should be interpreted flexibly to include not only the numerical values explicitly recited as the limit of the range, but also to include all the individual numerical values or sub-ranges encompassed within that range as if each numerical value and sub-range is explicitly recited.

For example, a concentration range of 0.1 to 5 ng/ml should be interpreted to include not only the explicitly recited concentration limits of 0.1ng/ml and 5ng/ml but also to include individual concentrations such as 0.2 ng/ml, 0.8 ng/ml, 1.0 ng/ml, 2.2 ng/ml, 3.6 ng/mol, and sub-ranges such as about 03. ng/ml to about 2.5 ng/ml, about 1.8 ng/ml to about 3.2 ng/ml, etc. This interpretation should apply regardless of the breadth of the range or the characteristic being described.

"%" as used herein, refers to the percentage by weight of the total composition, unless otherwise specified. All percentages are based on the percent by weight of the final composition prepared unless otherwise indicated and all totals equal 100% by weight.

The term "wt/wt" or "by weight", unless otherwise indicated, means the weight of a given component or specified combination of components to the total weight of the composition expressed as a percentage.

In one embodiment, the term "pharmaceutically acceptable" means approvable by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the therapeutic agent is administered.

The term "pharmaceutically acceptable excipient" means a pharmaceutically acceptable material, composition or vehicle involved in giving form or consistency to the pharmaceutical composition. Each excipient must be compatible with the other ingredients of the pharmaceutical composition when commingled such that interactions which would substantially reduce the efficacy of the compound of the invention when administered to an individual and interactions which would result in pharmaceutical compositions that are not pharmaceutically acceptable are avoided. In addition, each excipient must of course be of sufficiently high purity to render it pharmaceutically acceptable.

It should be understood that the terms "a" and "an" as used herein refer to "one or more" of the enumerated components. It will be clear to one of ordinary skill in the art that the use of the singular includes the plural unless specifically stated otherwise.

The term "and/or" as used herein covers both additively and also alternatively the individual elements of a list which are thus linked so that these elements are to be understood as linked selectively with "and" or respectively with "or". Furthermore, the terms used in the singular of course also comprise the plural.

Throughout the application, descriptions of various embodiments use "comprising" language, however in some specific instances, an embodiment can alternatively be described using the language "consisting essentially of' or "consisting of'.

"Substantially free" of a specified component refers to a composition with less than about 1% by weight of the specified component. "Free" of a specified component refers to a composition where the specified component is absent.

As used herein "mammal" includes but is not limited to humans, including pediatric, adult and geriatric patients.

The present invention also provides for use of a pharmaceutical composition for use in the topical treatment of vitiligo. Such compositions comprise an effective amount of the compound or a pharmaceutically acceptable salt thereof, and a pharmaceutically acceptable carrier.

The present invention also provides for a pharmaceutical product comprising a combination of therapeutic agents, for simultaneous, separate or sequential use in the treatment of conditions for which administration of the first therapeutic agent is indicated.

In one aspect of the invention, the vitiligo of a subject in need thereof is treated using an effective amount of 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof, in combination or adjunctively with a second therapeutic treatment or active agent.

Another aspect of the invention, the vitiligo of a subject in need thereof is treated using an effective amount of 7-(2-(methyloxy)-6-{[(4-methyl-2-pyridinyl)methyl]oxy}phenyl)-2,3,4,5-tetrahydro-1*H*-3-benzazepine, mesylate in combination or adjunctively with a second therapeutic treatment or active agent.

In one aspect of the invention the vitiligo of a subject in need thereof is treated using an effective amount of 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof in combination or adjunctively with a second therapeutic treatment or active agent.

The compound used to treat vitiligo may be administered directly to the skin. The compound can be administered as a medicament by being compounded into a pharmaceutically or dermatologically acceptable composition or formulation. These compositions or formulations may include any of the various known excipients which may be applied topically and which will permit even spreading of the active ingredient over the affected area, rapid drying, and/or increased penetration. Examples of suitable formulations can include solutions, creams, ointments, gels, lotions, sprays, aerosols, foam, powders, or suspensions.

Pharmaceutical compositions comprising the compound described herein may be manufactured by means of conventional mixing, dissolving, granulating, emulsifying, encapsulating, entrapping or by lyophilization processes. The compositions may be formulated in a conventional manner using one or more physiologically acceptable carriers, diluents, excipients or auxiliaries which facilitate processing of the compound into preparations which can be used locally or topically.

The compound(s) of the invention may be formulated into the pharmaceutical composition as a free base, a hydrate, or solvate, a pharmaceutically acceptable salt or a hydrate or a solvate of the salt. Typically, such salts are more soluble in aqueous solutions than the corresponding free acids and bases, but salts having lower solubility than the corresponding free acids and bases may also be formed.

In one embodiment, the pharmaceutical formulation comprises a compound of the invention and a pharmaceutically acceptable excipient, and optionally a diluent, antioxidant, preservative, gelling agent, pH adjusting agent, or stabilizer, or mixtures thereof suitably adapted for topical administration to the skin or the eye of a patient.

A person of skill in the art will appreciate that a pharmaceutically effective amount of the compound may vary, but typically a concentration ranging from 0.0001% to about 10% or more by weight (w/w) will provide a pharmaceutically effective amount of a compound of Formula (I) to the subject. In certain formulations, the pharmaceutically effective amount of the compound is from about 0.01% to about 10% (w/w), or from about 0.1% to about 10%(w/w). In other examples, the compound is present in at least 0.1%, 0.5%, 1% 2%, 3% or 5% (w/w).

For prophylactic administration, the compound may be administered to a patient at risk of developing vitiligo, such as those with a family history of vitiligo or premature graying of the hair. A suitable dosage range for prophylactic usage is also believed to be in a concentration ranging from 0.0001% to about 10% or more by weight (w/w). Other risk factors for prophylactic administration can be identified by those of skill in the art and may include subject's having Addison's disease, hypothyroidism, hyperthyroidism, alopecia greata, pernicious anemia, psoriasis, or adult-onset diabetes. Alternatively, prophylactic administration may be applied to avoid the progression of symptoms in a patient diagnosed with vitiligo.
The amount of compound administered will depend upon a variety of factors, including, for example, the particular type of vitiligo being treated, the mode of administration, the severity of the vitiligo, the age and weight of the patient, the bioavailability of the particular active compound, etc. Determination of an effective dosage is well within the capabilities of those skilled in the art. A skilled practitioner will be able to determine the optimal dose for a particular individual. Effective dosages may be estimated initially from *in vitro* assays. For example, an initial dosage for use in animals may be formulated to achieve a skin (epidermal and/or dermal) concentration of active compound that is at or above an IC₅₀ of the particular compound as measured in an in vitro assay. Calculating dosages to achieve such skin concentrations taking into account the bioavailability of the particular compound is well within the capabilities of skilled artisans.

Initial dosages can also be estimated from *in vivo* data, using animal models. Animal models useful for testing the efficacy of compounds to treat or prevent the various diseases described above are well-known in the art. The compounds may be administered once per week, several times per week (for example, every other day), once per day or multiple times per day, depending upon the judgment of the prescribing physician.

For topical or ocular administration, effective dosages may be those where no significant systemic circulation of the compounds results from administration to the skin or eye, for example, where a topical formulation is applied directly to a depigmented patch and a localized dose is utilized prior to significant systemic circulation.

In certain examples an ophthalmic composition containing a compound of formula (I) for ocular administration (e.g., for vitiligo of the eye) may include a tonicity agent, a buffer, or both. In certain examples of ophthalmic compositions the tonicity agent is a simple carbohydrate or a sugar alcohol. As is known to one of ordinary skill in the art, tonicity agents may be used in the present compositions to adjust the tonicity of the composition, preferably to that of normal tears. Examples of suitable tonicity agents include, without limitation sodium chloride, potassium chloride, magnesium chloride, calcium chloride, carbohydrates, such as dextrose, fructose, galactose, polyols, such as sugar alcohols, including by way of example, mannitol, sorbitol, xylitol, lactitol, isomalt, maltitol and combinations thereof. Compositions containing a buffer contain, in some examples, a phosphate, citrate, or both.

The pharmaceutical compositions for the administration of the compound may conveniently be presented in dosage unit form and may be prepared by any of the methods well known in the art. The pharmaceutical compositions may be, for example, prepared by uniformly and intimately bringing the active ingredient into association with a liquid carrier or a finely divided solid carrier or both, and then, if necessary, shaping the product into the desired formulation and/or placing it in appropriate packaging. In topical formulations of the disclosed compounds, the formulation may be placed in an appropriate container (such as a squeeze-tube with a cap for dispensing ointments and creams). Alternatively, the dispenser may include a device for dispensing unit dosages of the drug (such as a bottle or dropper that dispenses a controlled pre-determined dosage of the drug to a target area).

In addition to the composition being suitable for administration to the skin, the dosage form, such as solutions, gels, ointments, creams and suspensions, etc., of said compositions may further optionally include, but are not limited to, additional excipients such as preservatives, antioxidants, emollients, humectants, penetration enhancer, surfactants, emulsifiers, buffering agents, pH control agents, film forming agents, chelating agents, solvents and co-solvents.

In one embodiment, the formulation is a solution. In another embodiment, the formulation is a gel. In another embodiment, the formulation is a suspension. In yet another embodiment, the formulation is a cream or ointment. One embodiment is any of the aforementioned formulations in a kit for topical or local administration. In one embodiment, the formulation is a liquid, for example a homogeneous liquid or a suspension, sold in a bottle which dispenses the formulation as drops or a liquid film (for example from an applicator tip that contacts a target area of the skin to dispense the liquid substantially only on a target area of the skin to be treated). In one embodiment, the formulation is a cream or ointment, sold in a tube which dispenses the formulation to a target area of the skin. In another embodiment, the compound is provided in a viscous liquid for rubbing into the skin or instilling in the eye. The formulations may have preservatives or be preservative-free (for example in a single-use container).

The compound described herein, or compositions thereof, will generally be used in an amount effective to treat or prevent vitiligo.

When used to treat vitiligo patches of the skin and/or mucous membranes, the compound may be administered singly, as a mixture and/or in combination with a second therapeutic treatment or agents useful for treating diseases and/or disorders of the skin. Suitable agents for treatment include but are not limited to steroids, membrane stabilizers, anti-inflammatory agents such as corticosteroids, 5-lipoxygenase (5LO) inhibitors, leukotriene synthesis and receptor inhibitors, inhibitors of IgE isotype switching or IgE synthesis, IgG isotype switching or IgG synthesis, β-agonists, tryptase inhibitors, aspirin, cyclooxygenase (COX) inhibitors, methotrexate, anti-TNF drugs, biologic agents such as rituxan, PDE4 inhibitors, and p38 inhibitors.

Other therapeutic agents for use with the compound herein may include but are not limited to antihistamines, antibacterials, antiviral agents, antifungal, antipsoriasis medications, thyroid hormone replacement compounds, PDE4 inhibitors, topical immunomodulators (such as tacrolimus or pimecrolimus), or psoralen (a UV sensitizer used in conjunction with phototherapy) and antihistamines. Medicament for other conditions such as acne or warts, e.g. retinoids and salicylic acid are also contemplated in the scope of this invention.

In one embodiment the second therapeutic agent is a corticosteroid. Corticosteriods are grouped by potency, from Group I (very potent: up to 600 times stronger than hydrocortisone) to Group VII. All are included within the scope of this invention.

Suitably, Group I steroids include, but are not limited to: Clobetasol propionate 0.05% (Dermovate®), Betamethasone dipropionate 0.25% (Diprolene®), Halobetasol proprionate 0.05% (Ultravate®), and Diflorasone diacetate 0.05% (Psorcon®).

Group II steroids include, but are not limited to: Fluocinonide 0.05% (Lidex®), Halcinonide 0.05% (Halog®), Amcinonide 0.05% (Cyclocort®), and Desoximetasone 0.25% (Topicort®).

Group III steroids include, but are not limited to: Triamcinolone acetonide 0.5% (Kenalog®, Aristocort® cream), Mometasone furoate 0.1% (Elocon®ointment), Fluticasone propionate 0.005% (Cutivate®), and Betamethasone dipropionate 0.05% (Diprosone®).

Group IV steroids include, but are not limited to: Fluocinolone acetonide 0.01-0.2% (Synalar®, Synemol®, Fluonid®), Hydrocortisone valerate 0.2% (Westcort®), Hydrocortisone butyrate 0.1% (Locoid®), Flurandrenolide 0.05% (Cordran®), Triamcinolone acetonide 0.1% (Kenalog®, Aristocort®A ointment), and Mometasone furoate 0.1% (Elocon® cream, lotion)

Group V steroids include, but are not limited to: Triamcinolone acetonide 0.1% (Kenalog®, Aristocort® cream, lotion), Fluticasone propionate 0.05% (Cutivate® cream), Desonide 0.05% (Tridesilon®, DesOwen® ointment), Fluocinolone acetonide 0.025% (Synalar®, Synemol® cream), and Hydrocortisone valerate 0.2% (Westcort® cream).

Group VI steroids include, but are not limited to: Prednicarbate 0.05% (Aclovate®cream, ointment), Triamcinolone acetonide 0.025% (Aristocort®A cream, Kenalog®lotion), Fluocinolone acetonide 0.01% (Capex® shampoo, Dermasmooth®), and Desonide 0.05% (DesOwen® cream, lotion).

Group VII steroids include, but are not limited to: Hydrocortisone 2.5% (Hytone® cream, lotion, ointment), and Hydrocortisone 1% (many over-the-counter brands).

In one embodiment the second therapeutic active agent is an anti-inflammatory compound, an antihistamine compound, an antibiotic compound, an antiviral compound, thyroid hormone replacement compounds, or any combination thereof.

In another embodiment the second therapeutic treatment is irradiation, systemic phototherapy, psoralen photochemotherapy, or excimer laser therapy.

In yet another embodiment the second therapeutic treatment or active agent may be utilized in combination with a compound of Formula (I). In one embodiment the compound of Formula (I) is 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

In another embodiment the compound of formula (I) is 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof. In one embodiment the pharmaceutically acceptable salt is the mesylate salt.

The pharmaceutical compositions disclosed herein may also be co-administered (concurrently or sequentially) with a variety of other treatments that are not applied to the skin, for example treatments that are administered systemically, such as orally or parenterally. Examples of such systemic treatments include corticosteroids (such as Prednisone), antibiotics (such as erythromycin, tetracycline, and dicloxacillin), antifungal agents (such as ketoconazole and Diflucan®), antiviral agents (such as acyclovir, and famcyclovir), corticosteroids (such as prednisone, methylprednisolone and prednisolone), immunosuppressants (such as mercaptopurine, Cytoxan®, cyclophosphamide, azathioprine, methotrexate, or mycophenolate), calcineurin inhibitors (such as cyclosporine, sirolimus and tacrolimus), biologics (such as Rituxan®, Enbrel®, Humira®, Remicade ®, Stelara ®, and Amevive®), and/or thyroid hormone replacement therapies, inhibitors of inosine monophosphate dehydrogenase (IMPDH) (such as mycophenolate, mycophenolate mofetil, azathioprine).

These various agents may be used in accordance with their standard or common dosages, as specified in the prescribing information accompanying commercially available forms of the drugs (see also, the prescribing information in the 2006 Edition of The Physician's Desk Reference). Azathioprine is currently available from Salix Pharmaceuticals, Inc. under the brand name Azasan®; mercaptopurine is currently available from Gate Pharmaceuticals, Inc. under the brand name Purinethol®; prednisone and prednisolone are currently available from Roxane Laboratories, Inc.; methyl prednisolone is currently available from Pfizer; sirolimus (rapamycin) is currently available from Wyeth-Ayerst under the brand name Rapamune®; tacrolimus is currently available from Fujisawa under the brand name Prograf®; cyclosporine is current available from Novartis under the brand name Sandimmune® and Abbott under the brand name Gengraf ®; IMPDH inhibitors such as mycophenolate mofetil and mycophenolic acid are currently available from Roche under the brand name Cellcept® and Novartis under the brand name Myfortic®; azathioprine is currently available from GlaxoSmithKline under the brand name Imuran®; and antibodies are currently available from Ortho Biotech under the brand name Orthoclone®, Novartis under the brand name Simulect® (basiliximab) and Roche under the brand name Zenapax® (daclizumab).

In one embodiment, the compound may be administered either in combination or adjunctively with systemic phototherapy (e.g., narrow-band UV-B phototherapy, 310-315 nm), psoralen photochemotherapy (PUVA: psoralens) (e.g., 5-methoxypsoralen, 8-methoxypsoralen (0.1-0.3%), trimethylpsoralen) combined with UV-A light), excimer laser (308 nm) therapy. In some examples, topical tacrolimus (0.03-0.1%) ointment is combined with excimer laser therapy. Pimecrolimus (1%) cream may be combined with narrow-band UV-B treatment for vitiligo of the face. Vitamin D analogs (e.g., calcipotriol, tacalcitol) may be combined with narrow-band UV-B or PUVA treatment.

In another embodiment, the compound is administered either in combination or adjunctively with the therapies above and/or with a therapy for another disease.

In one embodiment, the compound is administered either in combination or adjunctively with an ophthalmic formulation of a drug such as an antihistamine, an antibiotic, an anti-inflammatory, an antiviral or a glaucoma medication for treating cases of vitiligo that primarily affect the eye or skin around the eye (such as the eyelids), and may be administered to or around the eye, for example in drops or ointments. When preparing these combination formulations, the compound may be combined with ophthalmic antibiotics (such as sulfacetamide, erythromycin, gentamicin, tobramycin, ciprofloxacin or ofloxacin); ophthalmic corticosteroids (such as prednisolone, fluorometholone or dexamethasone; ophthalmic non-steroidal anti-inflammatories (such as ibuprofen, diclofenac, ketorolac or flurbiprofen); ophthalmic antihistamines (such as livostin, patanol, cromolyn, alomide, or pheniramine); ophthalmic antiviral eye medications (such as triflurthymidine, adenine, arabinoside or idoxuridine); ophthalmic glaucoma medications (for example beta-blockers such as timolol, metipranolol, carteolol, betaxolol or levobunolol); ophthalmic prostaglandin analogues (such as latanoprost); ophthalmic cholinergic agonists (such as pilocarpine or carbachol); ophthalmic alpha agonists such as bromonidine or iopidine; ophthalmic carbonic anhydrase inhibitors (such as dorzolamide); and ophthalmic adenergic agonists (such as epinephrine or dipivefrin).
The following examples are illustrative of the present invention and are not intended to be limitations thereon.

Other terms as used herein are meant to be defined by their well-known meanings in the art.

### Biologic Examples

### Vitiligo Animal Models

This example describes the use of animal models to screen for treatments for vitiligo, including the selection of regimens for treatment, prevention and combination treatments. Animal models are used to test the compound, as well as combination formulations, such as those described herein. In particular a topical formulation containing a compound of Formula (I) is applied to the skin of the animal and the therapeutic response is assessed. After the formulation is administered to the animal, the skin is examined for evidence of decreased number or size of depigmented areas.

The C57BL/6J Ler-vit/vit mouse strain has been beneficial as a vitiligo research tool (Lerner et al., J. Invest. Dermatol. 87(3):299-304 (September 1986)). This strain arose from the C57BL/6J strain. The vitiligo mouse has congenital dorsal and ventral white spots as well as replacement of pigmented hairs by white hairs. The lack of pigment is due to absence of melanocytes from the epidermis and hair follicles.

### Alternative Mouse Model

Another mouse model is the C57BL/6-mi^{vit}/mi^{vit} mouse, which has a slowly progressing retinal degeneration with unevenly pigmented retinal pigment epithelium. See, e.g., Smith et al., Invest. Ophthalmol. & Vis. Sci. 35(10):3625-3632 (September 1994). Another alternative animal model is the Smyth line (SL) chicken developed by Dr. J. Robert Smyth, Jr. at the University of Massachusetts, Amherst, Mass. See, e.g., Shi et al., BMC Immunol. 13:18 (April 2012); Stepicheva et al., J. Immunol. 184:83.16 (2010).

In these models, the animal is exposed to the test agent, using different routes, dosages and regimens of administration. In particular examples, the drugs disclosed herein are applied topically to the areas that have been or will be tape stripped. Alternatively, the test drug is administered systemically. The drug is administered one or more times at fixed intervals prior to or following tape stripping (for example, daily following tape stripping or following the appearance of depigmented skin areas or depigmented hair patches). Drug response can be assessed by measuring such indicia of disease as the number, surface area or size of depigmented areas. Histological analysis of skin specimens is also performed to determine the number of melanocytes within the epidermis. Other histological findings may include an increased number of Langerhans' cells, epidermal vacuolization, thickening of the basement membrane, T-cell inflammatory infiltrate, and neural alterations (Montes et al., Int. J. Dermatol., 42(1):57-61 (January 2003).) Deposits of extracellular granular material and/or foci of vacuolar degeneration of basal and parabasal keratinocytes may be observed (Moellmann et al., J. Inv. Dermatol. 79:312-330 (1982).) Inflammatory vitiligo may present with psoriasiform hyperplasia, parakeratotic mounds, acanthosis with elongation of rete ridges, and/or dilated vessels (Verma, Dermatology Online Journal 11(3):13 (2005).)

Another mouse model of vitiligo using adoptive transfer of melanocyte specific CD8+ T cells into mice with increased epidermal melanocytes which results in black skin and black hair is available. (JE Harris et al., Society for Investigative Dermatology, pg 1869-18-(2012). Mechanistic studies in this model reveal that depigmentation requires IFN-γ, which induces the local accumulation of melanocyte-specific CD8⁺ T cells within the skin. These results support a critical role for IFN-g in vitiligo and suggest that it acts to recruit CD8⁺ T cells to the skin. The ability of antibody-mediated neutralization of IFN-γ to prevent CD8⁺ T-cell accumulation in the skin as well as depigmentation is useful.

A mouse model of vitiligo developed in the Harris Lab uses adoptive transfer of melanocyte specific CD8+ T cells into mice (Mehdi Rashighi et al. (2014) Depigmentation in a Mouse Model of Vitiligo. Sci Transl Med 6:1-10). Briefly, vitiligo is induced through adoptive transfer of PMEL CD8+ T cells. PMEL CD8+ T cells are isolated from the spleens of PMEL TCR transgenic mice through negative selection on microbeads (Miltenyi Biotec) according to the manufacturer's instructions. Purified CD8+ T cells (1×106) are injected intravenously into sublethally irradiated (500 rads 1 day before transfer) Krt14- Kitl^{∗} hosts (12 to 16 weeks of age). Recipient mice also receive intraperitoneal injection of 1 × 106 plaque-forming units of rVV-hPMEL (N. Restifo, National Cancer Institute, NIH) on the same day of transfer. Mice used as controls for vitiligo are sublethally irradiated but do not receive rVV-hPMEL or PMEL CD8+ T cell transfer. Vitiligo score is objectively quantified by an observer blinded to the experimental groups, using a point scale based on the extent of depigmentation at four easily visible locations, including the ears, nose, rear footpads, and tails as described previously. Each location is examined, and the extent of depigmentation is estimated as a percentage of the anatomic site; both left and right ears and left and right rear footpads are estimated together and therefore evaluated as single sites. Points are awarded as follows: no evidence of depigmentation (0%) receive a score of 0, >0 to 10% = 1 point, >10 to 25% = 2 points, >25 to 75% = 3 points, >75 to <100% = 4 points, and 100% = 5 points. The "vitiligo score" is the sum of the scores at all four sites, with a maximum score of 20 points.

### CXCL10 Assay

### Cell culture experiments

A suitable CXCL10 assay may be used as described in Braegelmann et al., Experimental Dermatology, 2016, 25: 375-379. This assay is also described herein as: Immortalized keratinocytes (HaCaTs), purchased from CLS Cell Lines Service GmbH (Eppelheim, Germany), are cultured in cell culture flasks (Greiner, Bio-one, Frickenhausen, Germany) with DMEM (Life Technologies, Carlsbad, USA) containing 4500 mg/l D-glucose and L-glutamine, without sodium pyruvate, supplemented with 100 U/ml penicillin-streptomycin and 10% FBS. Fresh medium is added every second day and cells are passaged every four days. For visualization of intracellular pSYK, these cells are cultivated on cover glasses and analyzed with immune-fluorescence.

### Cell stimulation and pSYK inhibition

Synthetic immuno-stimulatory nucleic acids Poly(I:C) (high molecular weight) and poly(dA:dT) are purchased from InvivoGen, San Diego, USA. Endogenous nucleic acids (eNA) are extracted from unstimulated HaCaT cells with the aid of the Genomic DNA from Tissue Kit, purchased from Macherey-Nagel (Dueren, Germany). In the case of poly(dA:dT) and eNA, Lipofectamine 2000 (Life Technologies, Carlsbad, USA) serves as a transfection reagent, poly(I:C) was added to the cultures alone. The agents are used in the following concentrations: poly(I:C) 20µg/ml, poly(dA:dT) 1µg/ml + Lipofectamine 2000, endogenous nucleic acids 5µg DNA/ml and 4µgRNA/ml + Lipofectamine 2000 which is respectively added at a concentration of 2.5 µl/ml; and a selective pSYK inhibitor, GSK143 is used at a final concentration of 3µM. GSK143 is described in WO2010/097248 and in Liddle et al., Bio & Med Chem Lett, 2011:21:6188-6194.

### SYK-Phosphorylation in cultivated HaCat-cells

PSYK immuno-flourescence staining is performed on cells cultured on cover glasses in Corning Costar cell culture plates ((CLS3513) Sigma-Aldrich, St. Louis, USA). The anti-pSYK 525/526 antibody (ab58575, Abcam, Cambridge, UK) is detected by anti-rabbit Rhodamine Red-X (711-295-152) (Jackson Immuno Research), and nuclear contrast staining is achieved with 4',6-diamidino-2-phenylindole (DAPI (D9542), Sigma-Aldrich, St. Louis, USA).Visualization is achieved with a high-resolution fluorescence microscope (Axio Observer Z1, from Zeiss, Germany).

The results are confirmed by Western Blotting. Whole protein concentration is determined using the Pierce BCA Protein Analysis Kit ((23227), Thermo Scientific, Waltham, USA). Samples are loaded onto polyacrylamide gels and then blotted onto nitrocellulose membranes. The levels of pSYK are then detected using the anti-SYK-525/526 antibody at a dilution of 1:140. The secondary antibody, donkey anti-rabbit IgG-HRP (sc-2313, Santa Cruz, Dallas, USA) is diluted 1:2000. SuperSignal West Dura Substrate (No. 34076, from Thermo Scientific, Waltham, USA) serves as a chemoluminescence. Loading controls are run with an anti-beta-Actin antibody (sc47778, from Santa Cruz, Dallas, USA).

Alternatively, immortalized keratinocytes (HaCaTs), purchased from CLS Cell Lines Service GmbH (Eppelheim, Germany), are cultured in cell culture flasks (Greiner, Bio-one, Frickenhausen, Germany) with DMEM (Life Technologies, Carlsbad, USA) containing 4500 mg/l D-glucose and L-glutamine, without sodium pyruvate, supplemented with 100 U/ml penicillin-streptomycin and 10% FBS. Fresh medium iss added every second day and cells are passaged every four days. For visualization of intracellular pSYK, these cells are cultivated on cover glasses and analyzed with immune-fluorescence.

### Protein expression of pSYK regulated proteins

CXCL9, CXCL10, and OAS2 expression levels are analyzed within the supernatants of cells cultured and stimulated in Corning Costar cell culture plates (CLS3524, from Sigma-Aldrich, St. Louis, USA). To determine the concentrations of CXCL9 and CXCL10 a cytometric bead array (Human Flex Sets 558280 & 558286, from BD, San Jose, USA) is used following the manufacturer's instructions. OAS2 protein expression are measured by the Human 2',5'-Oligoadenylate-Synthetase 2 (OAS2) ELISA Kit (Wuxi Donglin Sci&Tech Development Co, Wuxi/Jiangsu, China).

### Cell stimulation and pSYK inhibition

The synthetic immunostimulatory nucleic acids poly(I:C) (high molecular weight) and poly(dA:dT) were purchased from InvivoGen, San Diego, USA. Endogenous nucleic acids (eNA) were extracted from unstimulated HaCaT cells with the aid of the Genomic DNA from Tissue Kit, purchased from Macherey-Nagel (Dueren, Germany). In the case of poly(dA:dT) and eNA, Lipofectamine 2000 (from Life Technologies, Carlsbad, USA) served as transfection reagent, poly(I:C) was added to the cultures alone. A selective SYK inhibitor, such as described herein (and specifically GSK143) derives from GlaxoSmithKline (GSK, Stevenage, UK). The agents were used in the following concentrations: poly(I:C) 20µg/ml, poly(dA:dT) 1µg/ml + Lipofectamine 2000, endogenous nucleic acids 5µg DNA/ml and 4µgRNA/ml + Lipofectamine 2000. Lipofectamine 2000 was added at a concentration of 2.5 µl/ml. The pSYK inhibitor, GSK143, was used at a final concentration of 3µM.

### Primary normal human epidermal keratinocytes

First passage adult normal human epidermal keratinocytes (NHEKs, FC-0025), human epidermis equivalents (epiCS, CS-1001-7D) and the appropriate media (LM-0027 and CS-3050) are purchased from CellSystems (Troisdorf, Germany). NHEKs and epiCS are cultured according to the manufacturer's instructions.

In the second passage, poly(I:C), poly(dA:dT) + Lipofectamine and GSK143 are added to the NHEK cultures as described for HaCaTs. The cultures are stimulated for 24 hours and CXCL10 was determined in the supernatant using the above mentioned Flex Set.

EpiCS are stimulated 6 days after arrival. Poly(I:C) and the selective SYK inhibitor, such as GSK143, are administered in the same concentrations as for the other functional experiments. 24 hours after stimulation, epiCS are formalin fixed for 1 hour, paraffin embedded and then processed for immunohistochemistry as described above. All experiments were conducted in triplicate.

In another CXCL10 assay, immortalized keratinocytes (HaCaTs) cells (AddexBio #T0020001) were cultured in DMEM (Thermo Fisher #10566-016) supplemented with 10% FBS and 1% penicillin/streptomycin. 1x10⁵ HaCaT cells were seeded per well of a 24-well culture dish in 400 µl culture medium. Once confluent, medium was aspirated and cells were pretreated with 200 µl of DMSO vehicle (0.1%) or 3-fold serial dilutions (10, 3.3, and 1.1 µM) of 7-(2-(methyloxy)-6-{[(4-methyl-2-pyridinyl)methyl]oxy}-phenyl)-2,3,4,5-tetrahydro-1*H*-3-benzazepine (Compound 1) for 30 minutes at 37°C with 5% CO2. After pre-treatment, 200 µl of poly(I:C) at 40 µg/ml was added per well (without removal of pre-treatment medium), making the final treatment concentration 20 µg/ml. Additional compound and/or DMSO was included to maintain concentrations. All samples received 0.1% DMSO during both pre-treatment and treatment phases. Cells were returned to the incubator for 48 hours. Each condition had biological triplicates.

At 48 hours, supernatants were collected, centrifuged to pellet debris, and then recollected. CXCL10 secreted into the supernatant was measured by the Human CXCL10/IP-10 Quantikine ELISA Kit (R&D Systems #DIP100) according to the manufacturer's protocol. 3 experiments were performed using identical methods, and the results were pooled as a percentage of maximum, with poly(I:C) + DMSO vehicle serving as the 100% control. All untreated samples that did not receive poly(I:C) (not shown) had undetectable CXCL10 levels (below 15.6 pg/ml).

### Results

Syk inhibition with 7-(2-(methyloxy)-6-{[(4-methyl-2-pyridinyl)methyl]oxy}-phenyl)-2,3,4,5-tetrahydro-1*H*-3-benzazepine (Compound 1) caused a dose-dependent decrease in CXCL10 secretion induced by poly(I:C) treatment of HaCaT cells **(****Figure 1****)** with maximal inhibition observed at the 10 µM dose concentration. The graph represents mean percentage of maximum stimulation (vehicle condition set to 100%) ± SEM of the 3 independent experiments pooled together. Within each experiment, the biological triplicate values were averaged to a single mean value for each condition. Graphs were made with GraphPad Prism 6.0.

### Methods of Treatment

Subjects to be treated with the pharmaceutical composition of the compound are selected based on a clinical and/or histopathological presentation of vitiligo. Subjects also may be selected based on an increased risk of developing vitiligo, such as a family history of vitiligo and/or presence of another disease associated with increased incidence of vitiligo. The compositions are generally applied topically to the depigmented areas on the skin, although they may also be applied more generally to the skin. Treatment may be continued for at least a week, month, or year.

A subject may also be selected for concomitant treatment with other pharmaceutical or non-pharmaceutical interventions, such as systemic Plaquenil®, topical corticosteroid, phototherapy, psoralen photochemotherapy, or excimer laser therapy. In other cases the compound may be administered with no other treatment for vitiligo.

Representative of a clinical trial treatment, a subject is selected by making a diagnosis of vitiligo in that patient. A pharmaceutically effective amount of the compound is provided in a topical formulation and the formulation is applied directly to depigmented areas, such as patches on the face and extremities. The pharmaceutical formulation is applied to the depigmented patches daily, for example 1-4 times per day for more than one day, for example at least one week. Topical application of the formulation to the depigmented areas is continued until the areas to which the formulation is applied show evidence of repigmentation or disappear, or their progression is delayed or stopped.

In other examples, the therapeutic compound is provided in an effective amount in a sunscreen formulation and is applied to the skin prior to exposure to ultraviolet radiation, to protect against exposure to ultraviolet radiation. The sunscreen formulation may contain, for example, an effective amount of an organic sunscreen, or an inorganic filter, such as titanium dioxide or zinc oxide, or a combination thereof to minimize skin exposure to ultraviolet radiation.

In some examples, the subject is diagnosed with a disorder in addition to vitiligo. For example, the subject presents with vitiligo and another pre-existing disorder. The subject may be treated with a topical formulation that contains the compound, or the subject may be treated in combination or adjunctively with a therapeutic agent for the pre-existing disorder. If the pre-existing disorder is, for example, a thyroid disorder, the subject may be treated with thyroid hormone replacement. If the pre-existing disorder is, for example, acne, the subject may be treated with a suitable acne agent.

In another example, a subject with vitiligo of the eye, or around the eye, may also be diagnosed with dry eyes and the combination therapy is administered to the subject. In one example, the subject is found to have a meibomitis that would be responsive to topical application of corticosteroids, such as a prednisolone acetate ophthalmic suspension 1%. The compound herein may be suspended in the prednisolone formulation and instilled in or applied to the eye 2 to 4 times a day or as determined. In other examples, if the dry eyes are associated with seasonal allergies or other inflammatory conditions, the eye drops may be administered with or in a formulation that includes antihistamines (such as pheniramine, emedastine, or azelastine), decongestants (such as tetrahydrozoline hydrochloride or naphazoline), or a non-steroidal anti-inflammatory agent (such as nepafenac or ketorolac), corticosteroids (such as fluorometholone or loteprednol), mast cell stabilizers (such as azelastie, cromal, emedastine, ketotifen, Iodoxamine, nedocromil, olopatadine, or pemirolast). If the dry eyes are associated with an infectious bacterial condition (such a meibomian gland infection or corneal infection) the eye drops may be administered with or in a combination formulation can contain appropriate antibiotics (such as ciprofloxacin, erythromycin, gentamicin, ofloxacin, sulfacetamine, tobramycin, or monofloxacin). If the dry eyes are associated with a viral infection, the eye drops may be administered with or in a combination formulation with an anti-viral agent such as trifluridine or idoxuridine.

The compositions of the invention may be used in an application device that permits application of the composition to a target site on the skin without applying the composition to non-target site areas of the skin. For example, a device may be employed that allows the composition to be applied without first applying the composition to one's fingers. Suitable devices include spatulas, swabs, syringes without needles, and adhesive patches. Use of spatulas or swabs, or the like may require the device to be inserted into a container containing the composition. Using syringes or adhesive patches may be accomplished by filling the syringe or patch with the composition. The composition may then be topically spread by the spatulas or swabs, or may be expelled from the syringes onto the person's skin.

In one embodiment of the invention, the composition containing the compound and an enhancing agent or penetration agent may be provided in an adhesive patch. Some examples of adhesive patches are well known. For example, see U.S. Pat. Nos. Des. 296,006; 6,010,715; 5,591,767; 5,008,110; 5,683,712; 5,948,433; and 5,965,154. Such patches generally have an adhesive layer, which is applied to a person's skin, a depot or reservoir for holding the pharmaceutical agent, and an exterior surface that prevents leakage of the pharmaceutical from the depot. The exterior surface of a patch is typically non-adhesive.

In accordance with the present invention, the compound for treating vitiligo may be incorporated into the patch so that the compound remains stable for extended periods of time. The compound may be incorporated into a polymeric matrix that stabilizes it, and permits the compound to diffuse from the matrix and the patch. The compound may also be incorporated into the adhesive layer of the patch so that once the patch is applied to the skin the compound may diffuse on to the skin or even into or through the skin.

Alternatively, the compound may be provided in one or more wells or pockets disposed near the surface of the patch that will contact the skin. In one embodiment, the compound is stored in the wells in a dried, or lyophilized state. Storing such patches in a cooled atmosphere (e.g., about 4 °C) maintains the stability of the compound. A patch may be removed from the cool atmosphere when needed, and applied to a person's skin where the compound may be solubilized upon mixing with fluid, such as water or saline. The fluid may be provided separately or as a component of the patch. For example, fluid may be provided on a person's skin so that when the patch containing the dried compound interacts with the fluid, the compound is exposed to the fluid and is solubilized. The solubilized compound may then be able to be absorbed by the skin. As another example, the patch may contain one or more wells or pockets to hold fluid in the patch. The fluid may be forced from the wells or pockets to cause the fluid to mix with the dried compound. For example, the fluid may be provided in a pocket in the patch, and in some embodiments contains an agent for enhancing or activating the compound. Pressure exerted on the patch causes the pocket to rupture and release the fluid so that it mixes with the dried compound. The composition containing the compound may thus diffuse out of the patch. In another example, a fluid such as a gel or creams that contains water may be applied to the skin at a target site. The patch containing the dried compound is then applied to the skin where the fluid mixes with the compound and the composition moves out of the patch and on to the skin.

In patches containing wells of dried compound, the wells are sealed so that the compound remains in the wells until the compound is administered. Accordingly, the wells are sealed with a membrane or film that prevents the compound from diffusing from the wells in the compound's dry state, but that permits the compound to diffuse from the wells when it is solubilized. The membrane may either be porous or nonporous.

Additionally, the transdermal patch may include a plurality of small needles that extend through the stratum corneum, but do not extend into the dermis to rupture blood vessels. The needles may be between 20 µm and 1 mm long when extending from the dermal surface of the patch. Thus, the needles extend through the stratum corneum, but terminate before the dermis where the capillary beds are located. The needles may be solid or hollow. Hollow needles may have a lumen extending along their length so that the composition can pass from the depot in the patch to the end of the needle in the epidermis. Solid needles may be used to permit the composition to diffuse along the outer surface of the needle into the epidermis.

In use, the topical applicator is adhesively applied to a target area of the skin that has one or more depigmented patches, and the applicator is left in place until the compound in the patch is administered. The topical applicator (such as a patch) provides sustained release of the drug over a prolonged period of time, such as several hours, or even at least a day or longer.

The above description fully discloses the invention including preferred embodiments thereof. Modifications and improvements of the embodiments specifically disclosed herein are within the scope of the invention in as far as they are covered by the scope of the claims. Without further elaboration, it is believed that one skilled in the art can, using the preceding description, utilize the present invention to its fullest extent. Therefore, the Examples herein are to be construed as merely illustrative and not a limitation of the scope of the present invention in any way. The embodiments of the invention in which an exclusive property or privilege is claimed are defined as follows.

## Claims

1. A compound of formula (I): 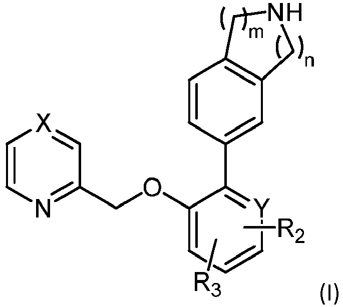 wherein:
X is CR₁ or N;
Y is CH, C or N;
R₁ is hydrogen, C₁₋₆alkoxy or C₁₋₆alkyl;
R₂ is hydrogen, C₁₋₆alkoxy, halo, -C(O)C₁₋₆alkyl, CN, Halo-C₁₋₆alkyl or C(O)NR₄R₅;
R₃ is hydrogen or C₁₋₆alkoxy;
R₄ is hydrogen or C₁₋₆alkyl:
R₅ is hydrogen or C₁₋₆alkyl and
m and n are integers each independently selected from 1 and 2; or a pharmaceutically acceptable salt thereof for use in the treatment of vitiligo or prevention of vitiligo in a subject at risk of developing vitiligo.

2. The compound for use according to claim 1 wherein X is CR₁.

3. The compound for use according to any one of claims 1 to 2 wherein R₂ is hydrogen, methoxy, fluoro or -C(O)CH₃.

4. The compound for use according to any one of claims 1 to 3 wherein R₃ is hydrogen, or methoxy.

5. The compound for use according to claim 1 wherein the compound of formula (1), or a pharmaceutically acceptable salt is
7-(3-{[(4-methyl-2-pyridinyl)methyl]oxy}-2-pyridinyl)-2,3,4,5-tetrahydro-1H-3-benzazepine;
7-(2-(methyloxy)-6-{[(4-methyl-2-pyridinyl)methyl]oxy}phenyl)-2,3,4,5-tetrahydro-1H-3-benzazepine;
7-(4-(methyloxy)-2-{[(4-methyl-2-pyridinyl)methyl]oxy}phenyl)-2,3,4,5-tetrahydro-1H-3-benzazepine;
1-[4-{[(4-methyl-2-pyridinyl)methyl]oxy}-3-(2,3,4,5-tetrahydro-1H-3-benzazepin-7-yl)phenyl]ethanone;
7-(6-methyl-3-{[(4-methyl-2-pyridinyl)methyl]oxy}-2-pyridinyl)-2,3,4,5-tetrahydro-1H-3-benzazepine;
1-[4-[(2-pyrazinylmethyl)oxy]-3-(2,3,4,5-tetrahydro-1H-3-benzazepin-7-yl)phenyl]ethanone;
7-(5-fluoro-2-{[(4-methyl-2-pyridinyl)methyl]oxy}phenyl)-1,2,3,4-tetrahydroisoquinoline;
7-(5-methyl-2-{[(4-methyl-2-pyridinyl)methyl]oxy}phenyl)-1,2,3,4-tetrahydroisoquinoline;
7-(2-(methyloxy)-6-{[(4-methyl-2-pyridinyl)methyl]oxy}phenyl)-1,2,3,4-tetrahydroisoquinoline;
7-(5-(ethyloxy)-2-{[(4-methyl-2-pyridinyl)methyl]oxy}phenyl)-1,2,3,4-tetrahydroisoquinoline;
7-(4-(methyloxy)-2-{[(4-methyl-2-pyridinyl)methyl]oxy}phenyl)-1,2,3,4-tetrahydroisoquinoline;
4-{[(4-methyl-2-pyridinyl)methyl]oxy}-3-(1,2,3,4-tetrahydro-7-isoquinolinyl)benzonitrile;
7-[2-{[(4-methyl-2-pyridinyl)methyl]oxy}-5-(trifluoromethyl)phenyl]-1,2,3,4-tetrahydroisoquinoline;
7-(5-(methyloxy)-2-{[(4-methyl-2-pyridinyl)methyl]oxy}phenyl)-1,2,3,4-tetrahydroisoquinoline;
7-(5-(1,1-dimethylethyl)-2-{[(4-methyl-2-pyridinyl)methyl]oxy}phenyl)-1,2,3,4-tetrahydroisoquinoline;
N-methyl-4-{[(4-methyl-2-pyridinyl)methyl]oxy}-3-(2,3,4,5-tetrahydro-1H-3-benzazepin-7-yl)benzamide;
4-{[(4-methyl-2-pyridinyl)methyl]oxy}-3-(2,3,4,5-tetrahydro-1H-3-benzazepin-7-yl)benzamide;
N,N-dimethyl-4-{[(4-methyl-2-pyridinyl)methyl]oxy}-3-(1,2,3,4-tetrahydro-7-isoquinolinyl)benzamide;
4-{[(4-methyl-2-pyridinyl)methyl]oxy}-3-(1,2,3,4-tetrahydro-7-isoquinolinyl)benzamide;
7-(2,3-bis(methyloxy)-6-{[(4-methy1-2-pyridiny1)methy1]oxy} pheny1)-1,2,3,4-tetrahydroisoquinoline;
7-(2,3-bis(methyloxy)-6-{[(4-methyl-2-pyridinyl)methyl]oxy}phenyl)-2,3,4,5-tetrahydro-1H-3-benzazepine;
7-[2-({[4-(methyloxy)-2-pyridinyl]methyl}oxy)phenyl]-2,3,4,5-tetrahydro-1H-3-benzazepine;
1-[4-[(2-pyridinylmethyl)oxy]-3-(2,3,4,5-tetrahydro-1H-3-benzazepin-7-yl)phenyl]ethanone, trifluoroacetate;
7-{6-methyl-3-[(2-pyrazinylmethyl)oxy]-2-pyridinyl}-2,3,4,5-tetrahydro-1H-3-benzazepine, trifluoroacetate;
7-(6-methyl-3-{[(4-methyl-2-pyridinyl)methyl]oxy}-2-pyridinyl)-2,3,4,5-tetrahydro-1H-3-benzazepine, trifluoroacetate;
7-{5-(methyloxy)-2-[(2-pyrazinylmethyl)oxy]phenyl}-2,3,4,5-tetrahydro-1H-3-benzazepine, trifluoroacetate;
7-[5-(methyloxy)-2-({[4-(methyloxy)-2-pyridinyl]methyl}oxy)phenyl]-2,3,4,5-tetrahydro-1H-3-benzazepine;
7-(5-(methyloxy)-2-{[(4-methyl-2-pyridinyl)methyl]oxy}phenyl)-2,3,4,5-tetrahydro-1H-3-benzazepine, trifluoroacetate;
1-[4-({[4-(methyloxy)-2-pyridinyl]methyl}oxy)-3-(2,3,4,5-tetrahydro-1H-3-benzazepin-7-yl)phenyl]ethanone;
1-[4-{[(4-methyl-2-pyridinyl)methyl]oxy}-3-(2,3,4,5-tetrahydro-1H-3-benzazepin-7-yl)phenyl]ethanone, trifluoroacetate;
7-[2-({[4-(methyloxy)-2-pyridinyl]methyl}oxy)phenyl]-2,3,4,5-tetrahydro-1H-3-benzazepine, trifluoroacetate;
1,1-dimethylethyl 7-[6-methyl-3-({[4-(methyloxy)-2-pyridinyl]methyl}oxy)-2-pyridinyl]-1,2,4,5-tetrahydro-3H-3-benzazepine-3-carboxylate;
7-(5-fluoro-2-{[(4-methyl-2-pyridinyl)methyl]oxy}phenyl)-2,3,4,5-tetrahydro-1H-3-benzazepine;
7-(5-methyl-2-{[(4-methyl-2-pyridinyl)methyl]oxy}phenyl)-2,3,4,5-tetrahydro-1H-3-benzazepine;
7-(5-(ethyloxy)-2-{[(4-methyl-2-pyridinyl)methyl]oxy}phenyl)-2,3,4,5-tetrahydro-1H-3-benzazepine;
7-(5-(methyloxy)-2-{[(4-methyl-2-pyridinyl)methyl]oxy}phenyl)-2,3,4,5-tetrahydro-1H-3-benzazepine;
4-{[(4-methyl-2-pyridinyl)methyl]oxy}-3-(2,3,4,5-tetrahydro-1H-3-benzazepin-7-yl)benzonitrile;
7-[2-{[(4-methyl-2-pyridinyl)methyl]oxy}-5-(trifluoromethyl)phenyl]-2,3,4,5-tetrahydro-1H-3-benzazepine;
7-(5-(1,1-dimethylethyl)-2-{[(4-methyl-2-pyridinyl)methyl]oxy}phenyl)-2,3,4,5-tetrahydro-1H-3-benzazepine;
7-(5-chloro-2-{[(4-methyl-2-pyridinyl)methyl]oxy}phenyl)-2,3,4,5-tetrahydro-1H-3-benzazepine;
7-(3-{[(4-ethyl-2-pyridinyl)methyl]oxy}-6-methyl-2-pyridinyl)-2,3,4,5-tetrahydro-1H-3-benzazepine;
7-(6-(1,1-dimethylethyl)-3-{[(4-methyl-2-pyridinyl)methyl]oxy}-2-pyridinyl)-2,3,4,5-tetrahydro-1H-3-benzazepine;
1-[4-{[(4-ethyl-2-pyridinyl)methyl]oxy}-3-(2,3,4,5-tetrahydro-1H-3-benzazepin-7-yl)phenyl]ethanone;
1-[4-({[4-(ethyloxy)-2-pyridinyl]methyl}oxy)-3-(2,3,4,5-tetrahydro-1H-3-benzazepin-7-yl)phenyl]ethanone;
1-[4-{[(4-{[2-(methyloxy)ethyl]oxy}-2-pyridinyl)methyl]oxy}-3-(2,3,4,5-tetrahydro-1H-3-benzazepin-7-yl)phenyl]ethanone;
7-{5-(methyloxy)-2-[(2-pyridinylmethyl)oxy]phenyl}-2,3,4,5-tetrahydro-1H-3-benzazepine;
1-[4-[(2-pyridinylmethyl)oxy]-3-(1,2,3,4-tetrahydro-7-isoquinolinyl)phenyl]ethanone;
7-{5-chloro-2-[(2-pyridinylmethyl)oxy]phenyl}-1,2,3,4-tetrahydroisoquinoline;
7-(6-chloro-3-{[(4-methyl-2-pyridinyl)methyl]oxy}-2-pyridinyl)-2,3,4,5-tetrahydro-1H-3-benzazepine;
7-(6-chloro-3-{[(4-methyl-2-pyridinyl)methyl]oxy}-2-pyridinyl)-1,2,3,4-tetrahydroisoquinoline;
1,1-dimethylethyl 5-{5-acetyl-2-[(2-pyridinylmethyl)oxy]phenyl}-1,3-dihydro-2H-isoindole-2-carboxylate;
1-[4-[(2-pyridinylmethyl)oxy]-3-(1,2,3,4-tetrahydro-6-isoquinolinyl)phenyl]ethanone; and
7-{2-(methyloxy)-6-[(2-pyrazinylmethyl)oxy]phenyl}-2,3,4,5-tetrahydro-1H-3-benzazepine, hydrochloride; or a salt thereof.

6. The compound for use according to claim 5 wherein the compound is 7-(2-(methyloxy)-6-{[(4-methyl-2-pyridinyl)methyl]oxy}phenyl)-2,3,4,5-tetrahydro-1*H*-3-benzazepine, or a pharmaceutically acceptable salt thereof; 7-(2-(methyloxy)-6-{[(4-methyl-2-pyridinyl)methyl]oxy}phenyl)-2,3,4,5-tetrahydro-1*H*-3-benzazepine, mesylate; 1-[4-{[(4-methyl-2-pyridinyl)methyl]oxy}-3-(2,3,4,5-tetrahydro-1*H*-3-benzazepin-7-yl)phenyl]ethanone, or a pharmaceutically acceptable salt thereof.

7. The compound for use according to any of the preceding claims, wherein the compound is administered as a topical formulation.

8. The compound for use according to any of the preceding claims, wherein the subject has one or more depigmented skin areas, and the compound is applied topically to at least one of the depigmented skin areas.

9. The compound for use according to any of claims 1 to 7, wherein the subject has vitiligo of the eyelid, and the compound is administered ocularly to the patient to treat the vitiligo.

10. The compound for use according to any of the preceding claims, wherein the compound is administered either in combination or adjunctively with a second therapeutic treatment or active agent.

11. The compound for use according to claim 10, wherein the second therapeutic active agent is an anti-inflammatory compound, an antihistamine compound, an antibiotic compound, an antiviral compound, thyroid hormone replacement compounds, or any combination thereof.

12. The compound for use according to claim 10, wherein the second therapeutic treatment is irradiation, systemic phototherapy, psoralen photochemotherapy, or excimer laser therapy.

13. The compound for use according to claim 12, wherein the second therapeutic treatment or active agent is utilized in combination with the compound.

## Patentansprüche

1. Eine Verbindung der Formel (I): 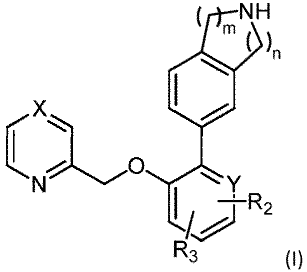 wobei:
X gleich CR₁ oder N ist;
Y gleich CH, C oder N ist;
R₁ Wasserstoff, C₁₋₆-Alkoxy oder C₁₋₆-Alkyl ist;
R₂ Wasserstoff, C₁₋₆-Alkoxy, Halogen, -C(O)C₁₋₆-Alkyl, CN, Halogen-C₁₋₆-alkyl oder C(O)NR₄R₅ ist;
R₃ Wasserstoff oder C₁₋₆-Alkoxy ist;
R₄ Wasserstoff oder C₁₋₆-Alkyl ist;
R₅ Wasserstoff oder C₁₋₆-Alkyl ist und
m und n ganze Zahlen sind, jeweils unabhängig ausgewählt aus 1 und 2; oder ein pharmazeutisch verträgliches Salz davon zur Verwendung bei der Behandlung der Weißfleckenkrankheit oder Vorbeugung der Weißfleckenkrankheit bei einem Individuum, bei dem ein Risiko zur Entwicklung der Weißfleckenkrankheit besteht.

2. Die Verbindung zur Verwendung nach Anspruch 1, wobei X gleich CR₁ ist.

3. Die Verbindung zur Verwendung nach einem der Ansprüche 1 bis 2, wobei R₂ Wasserstoff, Methoxy, Fluor oder -C(O)CH₃ ist.

4. Die Verbindung zur Verwendung nach einem der Ansprüche 1 bis 3, wobei R₃ Wasserstoff oder Methoxy ist.

5. Die Verbindung zur Verwendung nach Anspruch 1, wobei die Verbindung der Formel (1) oder ein pharmazeutisch verträgliches Salz
7-(3-{[(4-Methyl-2-pyridinyl)methyl]oxy}-2-pyridinyl)-2,3,4,5-tetrahydro-1H-3-benzazepin;
7-(2-(Methyloxy)-6-{[(4-methyl-2-pyridinyl)methyl]oxy}phenyl)-2,3,4,5-tetrahydro-1H-3-benzazepin;
7-(4-(Methyloxy)-2-{[(4-methyl-2-pyridinyl)methyl]oxy}phenyl)-2,3,4,5-tetrahydro-1H-3-benzazepin;
1-[4-{[(4-Methyl-2-pyridinyl)methyl]oxy}-3-(2,3,4,5-tetrahydro-1H-3-benzazepin-7-yl)phenyl]ethanon;
7-(6-Methyl-3-{[(4-methyl-2-pyridinyl)methyl]oxy}-2-pyridinyl)-2,3,4,5-tetrahydro-1H-3-benzazepin;
1-[4-[(2-Pyrazinylmethyl)oxy]-3-(2,3,4,5-tetrahydro-1H-3-benzazepin-7-yl)phenyl]ethanon;
7-(5-Fluor-2-{[(4-methyl-2-pyridinyl)methyl]oxy}phenyl)-1,2,3,4-tetrahydroisochinolin;
7-(5-Methyl-2-{[(4-methyl-2-pyridinyl)methyl]oxy}phenyl)-1,2,3,4-tetrahydroisochinolin;
7-(2-(Methyloxy)-6-{[(4-methyl-2-pyridinyl)methyl]oxy}phenyl)-1,2,3,4-tetrahydroisochinolin;
7-(5-(Ethyloxy)-2-{[(4-methyl-2-pyridinyl)methyl]oxy}phenyl)-1,2,3,4-tetrahydroisochinolin;
7-(4-(Methyloxy)-2-{[(4-methyl-2-pyridinyl)methyl]oxy}phenyl)-1,2,3,4-tetrahydroisochinolin;
4-{[(4-Methyl-2-pyridinyl)methyl]oxy}-3-(1,2,3,4-tetrahydro-7-isochinolinyl)benzonitril;
7-[2-{[(4-Methyl-2-pyridinyl)methyl]oxy}-5-(trifluormethyl)phenyl]-1,2,3,4-tetrahydroisochinolin;
7-(5-(Methyloxy)-2-{[(4-methyl-2-pyridinyl)methyl]oxy}phenyl)-1,2,3,4-tetrahydroisochinolin;
7-(5-(1,1-Dimethylethyl)-2-{[(4-methyl-2-pyridinyl)methyl]oxy}phenyl)-1,2,3,4-tetrahydroisochinolin;
N-Methyl-4-{[(4-methyl-2-pyridinyl)methyl]oxy}-3-(2,3,4,5-tetrahydro-1H-3-benzazepin-7-yl)benzamid;
4-{[(4-Methyl-2-pyridinyl)methyl]oxy}-3-(2,3,4,5-tetrahydro-1H-3-benzazepin-7-yl)benzamid;
N,N-Dimethyl-4-{[(4-methyl-2-pyridinyl)methyl]oxy}-3-(1,2,3,4-tetrahydro-7-isochinolinyl)benzamid;
4-{[(4-Methyl-2-pyridinyl)methyl]oxy}-3-(1,2,3,4-tetrahydro-7-isochinolinyl)benzamid;
7-(2,3-Bis(methyloxy)-6-{[(4-methyl-2-pyridinyl)methyl]oxy}phenyl)-1,2,3,4-tetrahydroisochinolin;
7-(2,3-Bis(methyloxy)-6-{[(4-methyl-2-pyridinyl)methyl]oxy}phenyl)-2,3,4,5-tetrahydro-1H-3-benzazepin;
7-[2-({[4-(Methyloxy)-2-pyridinyl]methyl}oxy)phenyl]-2,3,4,5-tetrahydro-1H-3-benzazepin;
1-[4-[(2-Pyridinylmethyl)oxy]-3-(2,3,4,5-tetrahydro-1H-3-benzazepin-7-yl)phenyl]ethanon, trifluoracetat;
7-{6-Methyl-3-[(2-pyrazinylmethyl)oxy]-2-pyridinyl}-2,3,4,5-tetrahydro-1H-3-benzazepin, trifluoracetat;
7-(6-Methyl-3-{[(4-methyl-2-pyridinyl)methyl]oxy}-2-pyridinyl)-2,3,4,5-tetrahydro-1H-3-benzazepin, trifluoracetat;
7-{5-(Methyloxy)-2-[(2-pyrazinylmethyl)oxy]phenyl}-2,3,4,5-tetrahydro-1H-3-benzazepin, trifluoracetat;
7-[5-(Methyloxy)-2-({[4-(methyloxy)-2-pyridinyl]methyl}oxy)phenyl]-2,3,4,5-tetrahydro-1H-3-benzazepin;
7-(5-(Methyloxy)-2-{[(4-methyl-2-pyridinyl)methyl]oxy}phenyl)-2,3,4,5-tetrahydro-1H-3-benzazepin, trifluoracetat;
1-[4-({[4-(Methyloxy)-2-pyridinyl]methyl}oxy)-3-(2,3,4,5-tetrahydro-1H-3-benzazepin-7-yl)phenyl]ethanon;
1-[4-{[(4-Methyl-2-pyridinyl)methyl]oxy}-3-(2,3,4,5-tetrahydro-1H-3-benzazepin-7-yl)phenyl]ethanon, trifluoracetat;
7-[2-({[4-(Methyloxy)-2-pyridinyl]methyl}oxy)phenyl]-2,3,4,5-tetrahydro-1H-3-benzazepin, trifluoracetat;
1,1-Dimethylethyl 7-[6-methyl-3-({[4-(methyloxy)-2-pyridinyl]methyl}oxy)-2-pyridinyl]-1,2,4,5-tetrahydro-3H-3-benzazepin-3-carboxylat;
7-(5-Fluor-2-{[(4-methyl-2-pyridinyl)methyl]oxy}phenyl)-2,3,4,5-tetrahydro-1H-3-benzazepin;
7-(5-Methyl-2-{[(4-methyl-2-pyridinyl)methyl]oxy}phenyl)-2,3,4,5-tetrahydro-1H-3-benzazepin;
7-(5-(Ethyloxy)-2-{[(4-methyl-2-pyridinyl)methyl]oxy}phenyl)-2,3,4,5-tetrahydro-1H-3-benzazepin;
7-(5-(Methyloxy)-2-{[(4-methyl-2-pyridinyl)methyl]oxy}phenyl)-2,3,4,5-tetrahydro-1H-3-benzazepin;
4-{[(4-Methyl-2-pyridinyl)methyl]oxy}-3-(2,3,4,5-tetrahydro-1H-3-benzazepin-7-yl)benzonitril;
7-[2-{[(4-Methyl-2-pyridinyl)methyl]oxy}-5-(trifluormethyl)phenyl]-2,3,4,5-tetrahydro-1H-3-benzazepin;
7-(5-(1,1-Dimethylethyl)-2-{[(4-methyl-2-pyridinyl)methyl]oxy}phenyl)-2,3,4,5-tetrahydro-1H-3 -benzazepin;
7-(5-Chlor-2-{[(4-methyl-2-pyridinyl)methyl]oxy}phenyl)-2,3,4,5-tetrahydro-1H-3-benzazepin;
7-(3-{[(4-Ethyl-2-pyridinyl)methyl]oxy}-6-methyl-2-pyridinyl)-2,3,4,5-tetrahydro-1H-3-benzazepin;
7-(6-(1,1-Dimethylethyl)-3-{[(4-methyl-2-pyridinyl)methyl]oxy}-2-pyridinyl)-2,3,4,5-tetrahydro-1H-3-benzazepin;
1-[4-{[(4-Ethyl-2-pyridinyl)methyl]oxy}-3-(2,3,4,5-tetrahydro-1H-3-benzazepin-7-yl)phenyl]ethanon;
1-[4-({[4-(Ethyloxy)-2-pyridinyl]methyl}oxy)-3-(2,3,4,5-tetrahydro-1H-3-benzazepin-7-yl)phenyl]ethanon;
1-[4-{[(4-{[2-(Methyloxy)ethyl]oxy}-2-pyridinyl)methyl]oxy}-3-(2,3,4,5-tetrahydro-1H-3-benzazepin-7-yl)phenyl]ethanon;
7-{5-(Methyloxy)-2-[(2-pyridinylmethyl)oxy]phenyl}-2,3,4,5-tetrahydro-1H-3-benzazepin;
1-[4-[(2-Pyridinylmethyl)oxy]-3-(1,2,3,4-tetrahydro-7-isochinolinyl)phenyl]ethanon;
7-{5-Chlor-2-[(2-pyridinylmethyl)oxy]phenyl}-1,2,3,4-tetrahydroisochinolin;
7-(6-Chlor-3-{[(4-methyl-2-pyridinyl)methyl]oxy}-2-pyridinyl)-2,3,4,5-tetrahydro-1H-3-benzazepin;
7-(6-Chlor-3-{[(4-methyl-2-pyridinyl)methyl]oxy}-2-pyridinyl)-1,2,3,4-tetrahydroisochinolin;
1,1-Dimethylethyl 5-{5-acetyl-2-[(2-pyridinylmethyl)oxy]phenyl}-1,3-dihydro-2H-isoindol-2-carboxy lat;
1-[4-[(2-Pyridinylmethyl)oxy]-3-(1,2,3,4-tetrahydro-6-isochinolinyl)phenyl]ethanon; und
7-{2-(Methyloxy)-6-[(2-pyrazinylmethyl)oxy]phenyl}-2,3,4,5-tetrahydro-1H-3-benzazepin, hydrochlorid ist; oder ein Salz davon.

6. Die Verbindung zur Verwendung nach Anspruch 5, wobei die Verbindung 7-(2-(Methyloxy)-6-{[(4-methyl-2-pyridinyl)methyl]oxy}phenyl)-2,3,4,5-tetrahydro-1*H-*3-benzazepin oder ein pharmazeutisch verträgliches Salz davon; 7-(2-(Methyloxy)-6-{[(4-methyl-2-pyridinyl)methyl]oxy}phenyl)-2,3,4,5-tetrahydro-1*H-*3-benzazepin, mesylat; 1-[4-{[(4-Methyl-2-pyridinyl)methyl]oxy}-3-(2,3,4,5-tetrahydro-1*H*-3-benzazepin-7-yl)phenyl]ethanon oder ein pharmazeutisch verträgliches Salz davon ist.

7. Die Verbindung zur Verwendung nach einem der vorhergehenden Ansprüche, wobei die Verbindung als eine topische Formulierung verabreicht wird.

8. Die Verbindung zur Verwendung nach einem der vorhergehenden Ansprüche, wobei das Individuum eine oder mehrere depigmentierte Hautstellen aufweist, und die Verbindung topisch auf mindestens einer der depigmentierten Hautstellen aufgetragen wird.

9. Die Verbindung zur Verwendung nach einem der Ansprüche 1 bis 7, wobei das Augenlid des Individuums von der Weißfleckenkrankheit betroffen ist, und die Verbindung dem Patienten okular verabreicht wird, um die Weißfleckenkrankheit zu behandeln.

10. Die Verbindung zur Verwendung nach einem der vorhergehenden Ansprüche, wobei die Verbindung entweder in Kombination mit oder zusätzlich zu einer zweiten therapeutischen Behandlung oder einem Wirkstoff verabreicht wird.

11. Die Verbindung zur Verwendung nach Anspruch 10, wobei es sich bei dem zweiten therapeutischen Wirkstoff um eine antientzündliche Verbindung, eine Antihistaminverbindung, eine antibiotische Verbindung, eine antivirale Verbindung, Verbindungen zum Ersatz von Schilddrüsenhormon oder eine Kombination davon handelt.

12. Die Verbindung zur Verwendung nach Anspruch 10, wobei es sich bei der zweiten therapeutischen Behandlung um Bestrahlung, systemische Phototherapie, Psoralen-Photochemotherapie oder Excimerlaser-Therapie handelt.

13. Die Verbindung zur Verwendung nach Anspruch 12, wobei die zweite therapeutische Behandlung oder der Wirkstoff in Kombination mit der Verbindung verwendet wird.

## Revendications

1. Composé de formule (1) : 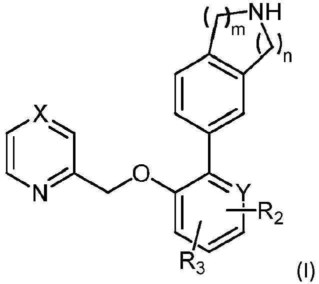 dans laquelle :
X est un groupe CR₁ ou N ;
Y est un groupe CH, C ou N ;
R₁ est un atome d'hydrogène, un groupe alkoxy en C₁ à C₆ ou alkyle en C₁ à C₆ ;
R₂ est un atome d'hydrogène, un groupe alkoxy en C₁ à C₆, halogéno, -C(O) (alkyle en C₁ à C₆), CN, halogénoalkyle en C₁ à C₆ ou C (O) NR₄R₅ ;
R₃ est un atome d'hydrogène ou un groupe alkoxy en C₁ à C₆ ;
R₄ est un atome d'hydrogène ou un groupe alkyle en C₁ à C₆ ;
R₅ est un atome d'hydrogène ou un groupe alkyle en C₁ à C₆ et
m et n sont des nombres entiers choisis chacun indépendamment parmi 1 et 2 ; ou un de ses sels pharmaceutiquement acceptables pour utilisation dans le traitement du vitiligo ou la prévention du vitiligo chez un sujet à risque de développer un vitiligo.

2. Composé pour utilisation selon la revendication 1, dans lequel X est un groupe CR₁.

3. Composé pour utilisation selon l'une quelconque des revendications 1 ou 2, dans lequel R₂ est un atome d'hydrogène, un groupe méthoxy, fluoro ou -C(O) CH₃.

4. Composé pour utilisation selon l'une quelconque des revendications 1 à 3, dans lequel R₃ est un atome d'hydrogène ou un groupe méthoxy.

5. Composé pour utilisation selon la revendication 1, où le composé de formule (I), ou un de ses sels pharmaceutiquement acceptables, est
la 7-(3-{[(4-méthyl-2-pyridinyl)méthyl]oxy}-2-pyridinyl)-2,3,4,5-tétrahydro-1H-3-benzazépine ;
la 7-(2-(méthyloxy)-6-{[(4-méthyl-2-pyridinyl)méthyl]-oxy}phényl)-2,3,4,5-tétrahydro-1H-3-benzazépine ;
la 7-(4-(méthyloxy)-6-{[(4-méthyl-2-pyridinyl)méthyl]-oxy}phényl)-2,3,4,5-tétrahydro-1H-3-benzazépine ;
la 1-[4-{[(4-méthyl-2-pyridinyl)méthyl]oxy}-3-(2,3,4,5-tétrahydro-1H-3-benzazépine-7-yl)phényl]éthanone ;
la 7-(6-méthyl-3-{[(4-méthyl-2-pyridinyl)méthyl]oxy}-2-pyridinyl)-2,3,4,5-tétrahydro-1H-3-benzazépine ;
la 1-[4-[(2-pyrazinylméthyl)oxy]-3-(2,3,4,5-tétrahydro-1H-3-benzazépine-7-yl)phényl]éthanone ;
la 7-(5-fluoro-2-{[(4-méthyl-2-pyridinyl)méthyl]oxy}-phényl)-1,2,3,4-tétrahydroisoquinoléine ;
la 7-(5-méthyl-2-{[(4-méthyl-2-pyridinyl)méthyl]oxy}-phényl)-1,2,3,4-tétrahydroisoquinoléine ;
la 7-(2-(méthyloxy)-6-{[(4-méthyl-2-pyridinyl)méthyl]-oxy}phényl)-1,2,3,4-tétrahydroisoquinoléine ;
la 7-(5-(éthyloxy)-2-{[(4-méthyl-2-pyridinyl)méthyl]-oxy}phényl)-1,2,3,4-tétrahydroisoquinoléine ;
la 7-(4-(méthyloxy)-2-{[(4-méthyl-2-pyridinyl)méthyl]-oxy}phényl)-1,2,3,4-tétrahydroisoquinoléine ;
le 4-{[(4-méthyl-2-pyridinyl)méthyl]oxy}-3-(1,2,3,4-tétrahydro-7-isoquinolinyl)benzonitrile ;
la 7-[2-{[(4-méthyl-2-pyridinyl)méthyl]oxy}-5-(trifluoro-méthyl)phényl]-1,2,3,4-tétrahydroisoquinoléine ;
la 7-(5-(méthyloxy)-2-{[(4-méthyl-2-pyridinyl)méthyl]-oxy}phényl)-1,2,3,4-tétrahydroisoquinoléine ;
la 7-(5-(1,1-diméthyléthyl)-2-{[(4-méthyl-2-pyridinyl)-méthyl]oxy}phényl)-1,2,3,4-tétrahydroisoquinoléine ;
le N-méthyl-4-{[(4-méthyl-2-pyridinyl)méthyl]oxy}-3-(2,3,4,5-tétrahydro-1H-3-benzazépine-7-yl)benzamide ;
le 4-{[(4-méhyl-2-pyridinyl)méthyl]oxy}-3-(2,3,4,5-tétrahydro-1H-3-benzazépine-7-yl)benzamide ;
le N,N-diméthyl-4-{[(4-méthyl-2-pyridinyl)méthyl]oxy}-3-(1,2,3,4-tétrahydro-7-isoquinolinyl)benzamide ;
le 4-{[(4-méthyl-2-pyridinyl)méthyl]oxy}-3-(1,2,3,4-tétrahydro-7-isoquinolinyl)benzamide ;
la 7-(2,3-bis(méthyloxy)-6-{[(4-méthyl-2-pyridinyl)-méthyl]oxy}phényl)-1,2,3,4-tétrahydroisoquinoléine ;
la 7-(2,3-bis(méthyloxy)-6-{[(4-méthyl-2-pyridinyl)-méthyl]oxy}phényl)-2,3,4,5-tétrahydro-1H-3-benzazépine ;
la 7-[2-({[(4-(méthyloxy)-2-pyridinyl]méthyl}oxy)phényl]-2,3,4,5-tétrahydro-1H-3-benzazépine ;
le trifluoroacétate de 1-[4-[(2-pyridinylméthyl)oxy]-3-(2,3,4,5-tétrahydro-1H-3-benzazépine-7-yl)phényl]éthanone ;
le trifluoroacétate de 7-{6-méthyl-3-[(2-pyrazinyl-méthyl)oxy]-2-pyridinyl}-2,3,4,5-tétrahydro-1H-3-benzazépine ;
le trifluoroacétate de 7-(6-méthyl-3-{[(4-méthyl-2-pyridinyl)méthyl]oxy}-2-pyridinyl)-2,3,4,5-tétrahydro-1H-3-benzazépine ;
le trifluoroacétate de 7-{5-(méthyloxy)-2-[(2-pyrazinyl-méthyl)oxy]phényl}-2,3,4,5-tétrahydro-1H-3-benzazépine ;
la 7-[5-(méthyloxy)-2-({[4-(méthyloxy)-2-pyridinyl)-méthyl}oxy)phényl]-2,3,4,5-tétrahydro-1H-3-benzazépine ;
le trifluoroacétate de 7-(5-(méthyloxy)-2-{[(4-méthyl-2-pyridinyl)méthyl]oxy}phényl)-2,3,4,5-tétrahydro-1H-3-benzazépine ;
la 1-[4-({[4-(méthyloxy)-2-pyridinyl]méthyl}oxy)-3-(2,3,4,5-tétrahydro-1H-3-benzazépine-7-yl)phényl]éthanone ;
le trifluoroacétate de 1-[4-{[(4-méthyl-2-pyridinyl)-méthyl]oxy}-3-(2,3,4,5-tétrahydro-1H-3-benzazépine-7-yl)-phényl]éthanone ;
le trifluoroacétate de 7-[2-({[4-(méthyloxy)-2-pyridinyl]-méthyl}oxy)phényl]-2,3,4,5-tétrahydro-1H-3-benzazépine ;
le 7-[6-méthyl-3-({[4-(méthyloxy)-2-pyridinyl]méthyl}-oxy)-2-pyridinyl]-1,2,4,5-tétrahydro-3H-3-benzazépine-3-carboxylate de 1,1-diméthyléthyle ;
la 7-{5-fluoro-2-{[(4-méthyl-2-pyridinyl)méthyl]oxy}-phényl)-2,3,4,5-tétrahydro-1H-3-benzazépine ;
la 7-{5-méthyl-2-{[(4-méthyl-2-pyridinyl)méthyl]oxy}-phényl)-2,3,4,5-tétrahydro-1H-3-benzazépine ;
la 7-(5-(éthyloxy)-2-{[(4-méthyl-2-pyridinyl)méthyl]-oxy}phényl)-2,3,4,5-tétrahydro-1H-3-benzazépine ;
la 7-(5-(méthyloxy)-2-{[(4-méthyl-2-pyridinyl)méthyl]-oxy}phényl)-2,3,4,5-tétrahydro-1H-3-benzazépine ;
le 4-{[(4-méthyl-2-pyridinyl)méthyl]oxy}-3-(2,3,4,5-tétrahydro-1H-3-benzazépine-7-yl)benzonitrile ;
la 7-{2-{[(4-méthyl-2-pyridinyl)méthyl]oxy}-5-(trifluoro-méthyl)phényl]-2,3,4,5-tétrahydro-1H-3-benzazépine ;
la 7-(5-(1,1-diméthyléthyl)-2-}[(4-méthyl-2-pyridinyl)-méthyl]oxy}phényl)-2,3,4,5-tétrahydro-1H-3-benzazépine ;
la 7-(5-chloro-2-{[(4-méthyl-2-pyridinyl)méthyl]oxy}-phényl)-2,3,4,5-tétrahydro-1H-3-benzazépine ;
la 7-(3-{[(4-méthyl-2-pyridinyl)méthyl]oxy}-6-méthyl-2-pyridinyl)-2,3,4,5-tétrahydro-1H-3-benzazépine ;
la 7-(6-(1,1-diméthyléthyl)-3-}[(4-méthyl-2-pyridinyl)-méthyl]oxy}-2-pyridinyl)-2,3,4,5-tétrahydro-1H-3-benzazépine ;
la 1-[4-{[(4-éthyl-2-pyridinyl)méthyl]oxy}-3-(2,3,4,5-tétrahydro-1H-3-benzazépine-7-yl)phényl]éthanone ;
la 1-[4-({[4-(éthyloxy)-2-pyridinyl]méthyl]oxy)-3-(2,3,4,5-tétrahydro-1H-3-benzazépine-7-yl)phényl]éthanone ;
la 1-[4-{[(4-{[2-(méthyloxy)éthyl]oxy}-2-pyridinyl)-méthyl]oxy}-3-(2,3,4,5-tétrahydro-1H-3-benzazépine-7-yl)-phényl]éthanone ;
la 7-{5-(méthyloxy)-2-[(2-pyridinylméthyl)oxy]phényl}-2,3,4,5-tétrahydro-1H-3-benzazépine ;
la 1-[4-[(2-pyridinylméthyl]oxy]-3-(1,2,3,4-tétrahydro-7-isoquinolinyl)phényl]éthanone ;
la 7-{5-chloro-2-[(2-pyridinylméthyl)oxy]phényl}-1,2,3,4-tétrahydroisoquinoléine ;
la 7-(6-chloro-3-{[(4-méthyl-2-pyridinyl)méthyl]oxy}-2-pyridinyl)-2,3,4,5-tétrahydro-1H-3-benzazépine ;
la 7-{6-chloro-3-{[(4-méthyl-2-pyridinyl)méthyl]oxy}-2-pyridinyl)-1,2,3,4-tétrahydroisoquinoléine ;
le 5-{5-acétyl-2-[(2-pyridinylméthyl)oxy]phényl}-1,3-dihydro-2H-isoindole-2-carboxylate de 1,1-diméthyléthyle ;
la 1-[4-[(2-pyridinylméthyl]oxy]-3-(1,2,3,4-tétrahydro-6-isoquinolinyl)phényl]éthanone ;
et
le chlorhydrate de 7-{2-(méthyloxy)-6-[(2-pyrazinyl-méthyl)oxy]phényl}-2,3,4,5-tétrahydro-1H-3-benzazépine ; ou un de ses sels.

6. Composé pour utilisation selon la revendication 5, où le composé est la 7-(2-(méthyloxy)-6-{[(4-méthyl-2-pyridinyl)méthyl]oxy}phényl)-2,3,4,5-tétrahydro-1H-3-benzazépine ou un de ses sels pharmaceutiquement acceptables ; le mésylate de 7-(2-méthyloxy)-6-{[(4-méthyl-2-pyridinyl)méthyl]-oxy}phényl)-2,3,4,5-tétrahydro-1H-3-benzazépine ; la 1-[4-{[(4-méthyl-2-pyridinyl)méthyl]oxy}-3-(2,3,4,5-tétrahydro-1*H*-3-benzazépine-7-yl)phényl]éthanone ou un de ses sels pharmaceutiquement acceptables.

7. Composé pour utilisation selon l'une quelconque des revendications précédentes, où le composé est administré comme une formulation topique.

8. Composé pour utilisation selon l'une quelconque des revendications précédentes, dans lequel le sujet a une ou plusieurs surfaces de peau dépigmentée, et le composé est appliqué topiquement sur au moins une des surfaces de peau dépigmentée.

9. Composé pour utilisation selon l'une quelconque des revendications 1 à 7, où le sujet a un vitiligo de la paupière, et le composé est administré par voie oculaire au patient pour traiter le vitiligo.

10. Composé pour utilisation selon l'une quelconque des revendications précédentes, où le composé est administré en combinaison ou conjointement avec un second traitement ou agent actif thérapeutique.

11. Composé pour utilisation selon la revendication 10, où le second agent actif thérapeutique est un composé anti-inflammatoire, un composé antihistaminique, un composé antibiotique, un composé antiviral, des composés de remplacement d'hormone thyroïdienne, ou l'une quelconque de leurs combinaisons.

12. Composé pour utilisation selon la revendication 10, où le second traitement thérapeutique est une exposition aux rayonnements, une photothérapie systémique, une photochimiothérapie du psoralène, ou une thérapie au laser d'excimère.

13. Composé pour utilisation selon la revendication 12, où le second traitement thérapeutique ou agent actif est utilisé en combinaison avec le composé.
